(19) Europäisches Patentamt
European Patent Office
Office européen des brevets

(11) EP 4 460 488 B1

(12) EUROPEAN PATENT SPECIFICATION

(45) Date of publication and mention of the grant of the patent:
**04.06.2025 Bulletin 2025/23**

(21) Application number: **24710125.6**

(22) Date of filing: **13.03.2024**

(51) International Patent Classification (IPC):
***C07C 309/15*** *(2006.01)*

(52) Cooperative Patent Classification (CPC):
(C-Sets available)
**C07C 309/15; C08F 20/58; C08F 220/56;**
C07B 2200/13 (Cont.)

(86) International application number:
**PCT/EP2024/056691**

(87) International publication number:
**WO 2024/189091 (19.09.2024 Gazette 2024/38)**

(54) **CRYSTALLINE FORM OF 2-ACRYLAMIDO-2-METHYLPROPANESULPHONIC ACID SODIUM SALT**

KRISTALLINE FORM DES NATRIUMSALZES VON 2-ACRYLAMIDO-2-METHYLPROPANSULFONSÄURE

FORME CRISTALLINE DE SEL DE SODIUM D'ACIDE 2-ACRYLAMIDO-2-MÉTHYLPROPANESULFONIQUE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **13.03.2023 FR 2302303**

(43) Date of publication of application:
**13.11.2024 Bulletin 2024/46**

(60) Divisional application:
**25172390.4**

(73) Proprietor: **SNF Group**
**42160 Andrézieux-Bouthéon (FR)**

(72) Inventors:
- **FAVERO, Cédrick**
**42160 ANDREZIEUX-BOUTHEON (FR)**
- **LEGRAS, Benoit**
**42160 ANDREZIEUX-BOUTHEON (FR)**
- **KIEFFER, Johann**
**42160 ANDREZIEUX-BOUTHEON (FR)**

(74) Representative: **Cabinet Laurent & Charras**
**Le Contemporain**
**50 Chemin de la Bruyère**
**69574 Dardilly Cedex (FR)**

(56) References cited:
**WO-A1-2014/117685**
**WO-A2-2013/079507**
**US-B1- 6 331 647**
**US-B2- 10 759 746**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C08F 220/56, C08F 220/585**

## Description

### Field of the invention

The present invention relates to a crystalline form of 2-acrylamido-2-methylpropanesulphonic acid. More specifically, the present invention relates to a crystalline form of 2-acrylamido-2-methylpropanesulphonic acid sodium salt. The invention also relates to the method for obtaining the crystalline form of 2-acrylamido-2-methylpropanesulphonic acid sodium salt.

### Prior art

2-acrylamido-2-methylpropanesulphonic acid, which is also referred to as ATBS, is widely used as an additive in acrylic fibers, or as a raw material for producing polymers used as dispersants, thickeners, friction reducers, flocculants or superabsorbents in various sectors including the oil and gas industry, mining, construction, textiles, water treatment (seawater desalination, mineral industry, etc.) or cosmetics.

The reaction carried out in the method for preparing ATBS corresponds to the reaction scheme below, in which acrylonitrile is present in excess so as to be both the reaction solvent and a reagent. The acrylonitrile is brought into contact with fuming sulphuric acid (oleum) and isobutylene.

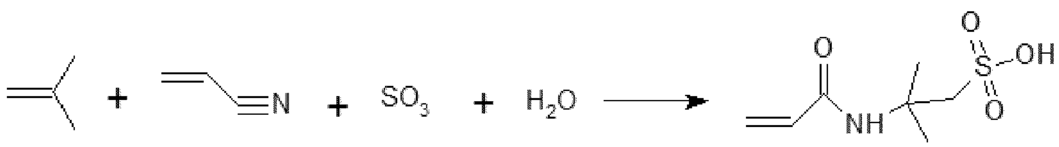

One by-product that may be produced during this synthesis is acrylamide.

ATBS is not soluble in acrylonitrile solvent. As a result, the reaction product is in the form of a suspension of crystals in the reaction solvent.

For example, documents US 6,448,347 and CN 102351744 describe a method for the continuous production of ATBS. The ATBS is then separated from the acrylonitrile, generally by filtration, and then dried.

It is necessary to dry ATBS in order to reduce the quantity of residual acrylonitrile and acrylamide present in the crystal. These two compounds are classified as carcinogenic, mutagenic or reprotoxic (CMR). It is therefore necessary to carry out effective filtration to dewater the acrylonitrile as thoroughly as possible, and then dry the ATBS in order to obtain low levels of acrylonitrile and acrylamide.

A person skilled in the art is aware that ATBS crystals have a crystallographic arrangement that produces a needle-shaped solid.

Needle-shaped crystals are known to a person skilled in the art to have macroscopic properties that pose difficulties in solid handling and transport operations (poor solid flowability, caking, low resistance to shear stress), and processing operations (poor filterability, drying difficulties, attrition).

In the case of ATBS, the additional problems encountered are generally the small particle size of the needle-shape crystals, the density of the solid in question, and the explosive nature of the fine dust.

These macroscopic properties are linked directly to the morphology of the crystals and to their specific surface area. Needle-shape crystals have a high specific surface area.

Patents WO 2009/072480, JP 2008/307822 and JP 2003/137857 describe that ATBS needle-shape crystals are obtained.

Document WO 2018/172676 filed by the Applicant describes a novel form of ATBS crystals referred to as a "hydrated crystalline form of ATBS". This novel crystalline form has physico-chemical properties that are different compared to the needle-shape ATBS and also gives improved properties to the polymers comprising ATBS in this novel form.

However, whatever the ATBS form, it remains a strong acid due to its sulphonic acid function, which is highly corrosive to metals. Due to the powdery nature of 2-acrylamido-2-methylpropanesulphonic acid powder, there is also a risk of chemical burns due to fine airborne particles coming into contact with the skin or eyes or being breathed into the lungs during powder handling operations.

When ATBS is used in a polymerization method, it must be in aqueous form. The aqueous phase may be used as it is, i.e., in acid form, or in a salt form obtained by reaction of the acid with an alkali metal, an alkaline-earth metal or a molecule containing an unsubstituted or substituted amine function.

The shelf life of this aqueous solution is generally short because of self-polymerization phenomena caused by exposure to temperature, UV light or pollutants such as iron or its oxidized forms, which can be produced by the corrosion of

metal pipes or containers caused by the acid form of ATBS. In addition, the increase in temperature generated by the self-polymerization of ATBS is well above the boiling point of water, which can lead to an increase in pressure in the container and cause an explosion. Therefore, self-polymerization phenomena present a certain risk to the safety of people and installations.

US 6331647 B1 and WO 2013/079507 A2 describe the preparation of the sodium salt of ATBS, as discussed below in comparative examples 2b and 2c.

The Applicant has discovered a novel form of ATBS referred to as a "crystalline form of the 2-acrylamido-2-methylpropanesulphonic acid sodium salt". This novel form offers improved physico-chemical and application properties (as with the "hydrated crystalline" form) while avoiding the intermediate step of forming the ATBS salt from the acid. The risks of burning, corrosion and self-polymerization are also reduced. Finally, the crystalline form of the sodium salt has a longer shelf life than an aqueous solution of the sodium salt of 2-acrylamido-2-methylpropanesulphonic acid.

The sodium salt of 2-acrylamido-2-methylpropanesulphonic acid is hereinafter referred to as ATBS.Na.

Using the crystalline form of ATBS.Na according to the invention is in line with the principle of environmental awareness and the impact of industry and mankind on the planet. The novel form of the product means that it is safer for handlers to use and reduces the energy impact due to the salification step (salifying ATBS) that is no longer necessary during the polymerization of 2-acrylamido-2-methylpropanesulphonic acid and due to the powder form, which allows more active ingredient to be transported (100% for the powder compared with a maximum of 50% for a solution). The improved shelf life of the product also means less waste resulting from the increased product dosage required as a result of the reduced performance of a product that is too old. Moreover, the improved performances of the polymers obtained from the crystalline form of the sodium salt of the invention help reduce the quantity of product necessary for the applications in which they are used, reducing the overall water consumption and emissions of greenhouse gases such as the $CO_2$.

## Disclosure of the invention

The object of the present invention is a specific form of 2-acrylamido-2-methylpropanesulphonic acid referred to hereinafter as a "crystalline form of the 2-acrylamido-2-methylpropanesulphonic acid sodium salt".

The present invention also relates to a method for producing the crystalline form of the 2-acrylamido-2-methylpropanesulphonic acid sodium salt (ATBS.Na).

## Description of the invention

The term "polymer" should be understood to mean a homopolymer or a copolymer. The term "copolymer" should be understood to mean a polymer obtained from at least two different monomers. It may therefore be a copolymer of at least two monomers chosen from hydrophilic anionic monomers, hydrophilic cationic monomers, hydrophilic non-ionic monomers, hydrophilic zwitterionic monomers, hydrophobic monomers and the mixtures thereof.

The term "hydrophilic monomer" should be understood to mean a monomer that has an octanol-water partition coefficient, $K_{ow}$, equal or less than 1, in which the partition coefficient $K_{ow}$ is determined at 25°C in an octanol-water mixture with a volume ratio of 1/1, at a pH of between 6 and 8.

The term "hydrophobic monomer" should be understood to mean a monomer that has an octanol-water partition coefficient, $K_{ow}$, greater than 1, in which the partition coefficient $K_{ow}$ is determined at 25°C in an octanol-water mixture with a volume ratio of 1/1, at a pH of between 6 and 8.

The term "crystal" or "crystalline form" refers to a solid material whose constituents (such as atoms, molecules, or ions) are arranged in a highly ordered microscopic structure, forming a crystal lattice that extends in all directions. It does not encompass amorphous solid.

The octanol-water partition coefficient, $K_{ow}$, represents the ratio of concentrations (g/L) of a monomer between the octanol phase and the aqueous phase. It is defined as follows:

$$K_{ow} = \frac{[monomer]_{octanol}}{[monomer]_{water}}$$

By definition, a water-soluble polymer is a polymer that gives an aqueous solution when it is dissolved while stirring at 25°C and with a concentration of 10 $g.L^{-1}$ in water.

"X and/or Y" should be understood to mean "X", or "Y", or "X and Y".

The invention also includes all possible combinations of the various embodiments disclosed, whether they are preferred embodiments or given by way of example. Furthermore, when ranges of values are indicated, the limit values are included in these ranges. The disclosure also includes all of the combinations between the limit values of these ranges of values. For example, the ranges of values "1-20, preferably 5-15" imply disclosure of the ranges "1-5", "1-15", "5-20" and

"15-20" and the values 1, 5, 15 and 20.

**Crystalline form of the sodium salt of 2-acrylamido-2-methylpropanesulphonic acid**

The present invention relates to a crystalline form of the 2-acrylamido-2-methylpropanesulphonic acid sodium salt having an X-ray powder diffraction pattern comprising peaks at 11.7°; 12,2°; 13,2°; 13,5°; 15,6°; 16,8°; 17,8°; 18,5°; 19,1°; 20,6°; 21,4°; 23,3°; 25,1°; 25,8°; 26,9°; 29,1°; 29,5°; 31,0°; 33,0°; 33,6°; 34,4°; 35,2°; 35,9°; 37,1°; 38,4°; 39,6°; 41,1°; 42,9°; 45,1°; 46,0°; 47,2°; 47,6 2-theta angles. The uncertainty of these peaks is generally of the order of +/- 0.1°.

X-ray crystallography, radiocrystallography or X-ray diffractometry is an analytical technique used to study the structure of crystalline matter on an atomic scale. It is based on the physical phenomenon of X-ray diffraction. A diffractometer with a copper source can be used.

A powder formed from a given crystalline phase always gives rise to diffraction peaks in the same directions. This diffraction pattern thus forms a true signature of the crystalline phase. It is therefore possible to determine the nature of each crystalline phase within a mixture or a pure product.

This signature is specific to each organic or inorganic compound, and is in the form of a list of peaks positioned at an angle of $2\theta$ (2-theta).

This technique is used to characterize matter, in particular the different crystalline forms that can exist for the same chemical molecule, also known as polymorphs.

Another aspect of the invention relates to a crystalline form of ATBS.Na having a Fourier-transform infrared spectrum comprising peaks at 3576 $cm^{-1}$, 3485 $cm^{-1}$, 3310 $cm^{-1}$, 3079 $cm^{-1}$, 2975 $cm^{-1}$, 1658 $cm^{-1}$, 1629 $cm^{-1}$, 1543 $cm^{-1}$, 1403 $cm^{-1}$, 1321 $cm^{-1}$, 1301 $cm^{-1}$, 1205 $cm^{-1}$, 1187 $cm^{-1}$, 1163 $cm^{-1}$, 1046 $cm^{-1}$, 980 $cm^{-1}$, 629 $cm^{-1}$. The uncertainty of these peaks is generally of the order of +/- 8 $cm^{-1}$.

The infrared measurement is carried out by Fourier transform, for example using a Perkin Elmer Spectrum 100 spectrometer fitted with a single reflection ATR polarization accessory, with an accuracy of 8 $cm^{-1}$.

Fourier-transform infrared spectroscopy is the analysis of the vibrations emitted, absorbed or scattered by molecules. This technique is sensitive to so-called short interactions (influence of the unit mesh on the bonds). In most cases, the Fourier-transform infrared spectra of different crystalline systems differ significantly. The Fourier-transform infrared spectrum therefore reflects the details of the crystalline structure of an organic compound.

Generally, and unless otherwise indicated, the X-ray diffraction pattern and the infrared spectrum are obtained at 20°C and at a pressure of 1 atmosphere absolute (101,325 Pa).

Another aspect of the invention relates to a crystalline form of ATBS.Na having a minimum ignition energy greater than 500 mJ, and preferably greater than 1000 mJ.

The minimum ignition energy represents the minimum energy that must be supplied to a compound to cause an ignite. The energy may be electrical or thermal. The minimum ignition energy is an essential piece of information when considering the risk of explosion during product handling (transfer, storage, reaction, shaping, etc.).

The minimum ignition energy depends on the properties of the powder (composition) and its macromolecular structure (particle size, crystalline form, specific surface area).

In the case of solids, this energy is the minimum energy of an electric spark likely to ignite a cloud of dust. The higher the value of the minimum ignition energy, the less risk the solid presents when used, handled or stored.

The minimum ignition energy is measured in accordance with standard NF EN 13821.

Another aspect of the present invention relates to a crystalline form of ATBS.Na exhibiting 4 thermal phenomena with the differential scanning calorimetry technique, at 49.8°C; 144.8°C; 169.8°C and 254.3°C. The uncertainty relating to the observation of these phenomena is generally of the order of 10°C (+/-10°C), advantageously 5°C or less.

The thermal phenomena are measured by differential scanning calorimetry (DSC). This technique uses the measurement of the variation in heat associated with the thermal denaturation of the compound when it is heated at constant speed, for example with a heating ramp of 10°C/minute.

**Method for producing the crystalline form of the sodium salt ATBS.Na**

The present invention also relates to the method for producing the crystalline form of ATBS.Na comprising at least the following successive steps:

1) mixing of ATBS with an aqueous solution $SA_1$ and at least one sodium salt base, advantageously for at least 1 minute, in order to form an aqueous solution or an aqueous suspension $SA_2$;
2) distillation, at a pressure of 700 mbar or less, of the aqueous solution $SA_2$ or of the aqueous suspension $SA_2$ in order to form a suspension $S_1$ and cooling of the aqueous solution or aqueous suspension $SA_2$ or a suspension $S_1$ wherein the temperature of the cooling step is decreased at a ramp of between 0.1 and 8°C/hour;;
3) solid-liquid separation of the suspension $S_1$ and isolation of the crystals of the suspension $S_1$ obtained at the end of

step 2) in the form of a composition $C_1$.

The crystals obtained are in the crystalline form of the sodium salt of 2-acrylamido-2-methylpropanesulphonic acid.

"Sodium salt(s) base" in step 1), should be understood to mean at least one inorganic sodium salt Brønsted base, for example sodium hydroxide, sodium carbonate, sodium bicarbonate or mixtures thereof.

The temperature and the mixing time of step 1) may vary as a function, in particular, of the concentration of ATBS. A person skilled in the art knows how to adapt the temperature and the mixing time to optimize the formation of the crystals.

The method for producing the crystalline form of ATBS.Na can be carried out on any form of ATBS, such as, for example, the needle-shape form or the hydrated form.

The production method may be carried out on ATBS of any degree of purity.

Therefore, the method may be carried out downstream of any type of method for producing ATBS. It may also be carried out on any forms of ATBS i.e. amorphous or crystalline that have already been obtained.

**Step 1) of the method for producing a crystalline form of ATBS.Na:**

ATBS is produced by a production method as previously described (acrylonitrile, fuming sulphuric acid and isobutylene). The ATBS may be in the form of fine powder or shaped in a controlled manner by a method such as compaction, granulation or extrusion.

The ATBS may be added to an aqueous solution SA1 before, after or in parallel with the sodium salt base, and preferably in parallel. Preferably, the aqueous solution SA1 is water.

The sodium salt base may be added as an aqueous solution, in this case, the aqueous solution of sodium salt base can be partially or totally the aqueous solution SA1.

Advantageously, the concentration of ATBS sodium salt in the aqueous solution or in the aqueous suspension $SA_2$ is between 1% by weight and saturation, preferably between 10% by weight and saturation, more preferably between 20% by weight and saturation, more preferably between 30% by weight and saturation, more preferably between 40% by weight and saturation, and even more preferably between 50% by weight and saturation, by weight relative to the weight of the aqueous solution or of the aqueous suspension $SA_2$.

The ATBS and the sodium base may be added all at once or in several stages. They are preferably added all at once.

When they are added in several stages, the ATBS and the sodium salt base are added in fractions.

When the ATBS and the sodium salt base are added in fractions, there is no limit to the number of fractions, advantageously there are at least two fractions, and preferably at least three fractions.

There is no limitation to the order of addition of the ATBS and the sodium salt base. They may be added at the same time (i.e., in parallel), one after the other (the ATBS first, then the sodium salt base, or vice versa), or alternately (a first fraction of ATBS, then a first fraction of the sodium salt base, followed by a second fraction of ATBS then a second fraction of the sodium salt base, and so on); they are preferably added at the same time.

When they are added one after the other or alternately, the second compound (whether it is the ATBS or the sodium salt base) can start being added before the first compound has finished being added.

A first fraction F1 of ATBS advantageously represents at least 1 mol% of the total of the ATBS present in the aqueous solution or the aqueous suspension $SA_2$, preferably at least 5 mol%, more preferably at least 10 mol%, even more preferably at least 15 mol% and even more preferably at least 20 mol%.

A second fraction F2 of ATBS advantageously represents at least 1 mol% of the total of the ATBS present in the aqueous solution or the aqueous suspension $SA_2$, preferably at least 5 mol%, more preferably at least 10 mol%, even more preferably at least 15 mol% and even more preferably at least 20 mol%.

A third fraction F3 of ATBS advantageously represents at least 1 mol% of the total of the ATBS present in the aqueous solution or the aqueous suspension $SA_2$, preferably at least 5 mol%, more preferably at least 10 mol%, even more preferably at least 15 mol% and even more preferably at least 20 mol%.

In one particular embodiment, the method is carried out continuously, in which case the ATBS and the sodium salt base are added continuously.

The quantity of ATBS in the aqueous solution or the aqueous suspension $SA_2$ is advantageously between 10 and 90% by weight relative to the total weight of the aqueous solution or of the aqueous suspension $SA_2$, preferably between 20 and 85% by weight, more preferably between 30 and 80% by weight.

The mixing of step 1) (ATBS + sodium salt base) is advantageously carried out at a temperature of between 0 and 90°C, preferably between 5 and 60°C, more preferably between 10 and 40°C, in order to obtain the aqueous solution or the aqueous suspension $SA_2$.

In one particular embodiment, the aqueous solution or the aqueous suspension $SA_2$ may comprise one or more organic solvents.

In some embodiments, the aqueous solution $SA_1$ may comprise one or more organic solvents.
The quantity of organic solvent may vary as a function of the temperature and the quantity of ATBS or sodium salt base. This quantity is not limited, provided it does not prevent the crystalline form of the sodium salt of ATBS from being obtained. A person skilled in the art knows how to determine this limit, which is a routine task. Generally, the aqueous solution or the aqueous suspension $SA_2$ comprises more water (by volume) than organic solvent.
The organic solvent or solvents are advantageously chosen from the following compounds:

- organic acids, advantageously carboxylic acids comprising between 1 and 8 carbon atoms;
- amides advantageously comprising between 1 and 8 carbon atoms;
- alcohols advantageously comprising between 1 and 8 carbon atoms;
- ketones advantageously comprising between 3 and 8 carbon atoms;
- ethers advantageously comprising between 2 and 8 carbon atoms;
- esters advantageously comprising between 2 and 8 carbon atoms;
- alkanes advantageously comprising between 4 and 8 carbon atoms, preferably between 5 and 6 carbon atoms;
- halogenated hydrocarbon compounds advantageously comprising between 1 and 8 carbon atoms;
- nitriles advantageously comprising between 1 and 8 carbon atoms; or
- mixtures thereof.

When an organic solvent is used in the invention, the temperature may be adjusted so that the solvent + water mixture remains in liquid form.
These compounds may be linear or branched. They may be saturated or comprise unsaturated bonds. An unsaturated bond corresponds to a double or triple bond (for example C=C or C≡C).
The organic solvent is preferably chosen from acrylonitrile, isopropanol, acrylic acid, acetic acid or mixtures thereof. The organic solvent is preferably acrylonitrile.
The organic solvent is generally in liquid form at the temperature at which steps 2) and 3) are carried out. Furthermore, it is advantageously partially miscible in water, and preferably completely miscible in water.
The organic solvent may, if necessary, be used to solubilize any impurities or by-products present with the ATBS used to form the aqueous solution or the aqueous suspension $SA_2$. However, ATBS is not necessarily soluble in the solvent.
In a preferred embodiment according to the invention, the aqueous solution or the aqueous suspension $SA_2$ does not contain organic solvent.
In a preferred embodiment according to the invention, the aqueous solution $SA_1$ does not contain organic solvent.
The time allowed for mixing the aqueous solution $SA_1$ and the ATBS is advantageously at least 1 minute, preferably between 1 minute and 600 minutes, more preferably between 5 minutes and 400 minutes, and even more preferably between 10 minutes and 240 minutes.
The compounds of step 1) can be mixed using various technologies. Examples include, but are not limited to, reactors with stirrers, loop reactors, static mixers, microreactors, piston reactors, agitated filter dryers, for example by Nutsche, paddle mixers, twin-cone mixers, ploughshare mixers and disc mixers.
The pH in step 1) is advantageously controlled between 6 and 14, preferably between 8 and 14, more preferably between 10 and 14, even more preferably between 12 and 14, and even more preferably between 13 and 14.
The quantity of ATBS.Na in the aqueous solution or the aqueous suspension $SA_2$ is advantageously between 10 and 90% by weight relative to the total weight of the aqueous solution or of the aqueous suspension $SA_2$, preferably 20 and 90%, preferably between 30 and 90% by weight, preferably between 50 and 90% by weight, preferably between 20 and 85% by weight, more preferably between 30 and 80% by weight.

**Step 2) of the method for producing the crystalline form of ATBS sodium salt:**

The distillation of the aqueous solution or of the aqueous suspension $SA_2$ takes place at a pressure of 700 mbar or less. It generally takes place in a vacuum distillation device, which is typically an evaporator. It is therefore also referred to here as "vacuum distillation".
When the aqueous solution or the aqueous suspension $SA_2$ is distilled, typically by passing it through an evaporator, crystals of the sodium salt of ATBS begins to form. Thus ATBS, the at least one sodium salt base, and crystalline solid particles of the sodium salt of ATBS coexists in the aqueous solution or the aqueous suspension $SA_2$.
The aqueous solution or the aqueous suspension $SA_2$ can be distilled using an evaporator. This may be a falling film evaporator, or a rising film evaporator, or a scraped thin film evaporator, or a short path evaporator, or a forced circulation evaporator, or a spiral tube evaporator, or a flash evaporator. It may also be a continuously stirred reactor. Preferably, distillation takes place in a scraped thin film evaporator, a short path evaporator or a forced circulation

evaporator. Even more preferably, distillation takes place in a scraped thin film evaporator.

Generally, an evaporator is a device comprising an inlet for solution to be treated (aqueous solution or aqueous suspension $SA_2$), an outlet for discharging the distilled solvent (the water and any organic solvents) and an outlet for discharging the suspension $S_1$.

The residence time of the aqueous solution or of the aqueous suspension $SA_2$ in the distillation device (advantageously under vacuum), which is advantageously an evaporator, in other words the distillation time at a pressure of 700 mbar or less, is advantageously comprised between 1 second and 600 seconds, preferably between 3 seconds and 300 seconds, more preferably between 30 seconds and 100 seconds. The residence time corresponds to the time necessary to carry out step 2), i.e., the time required to prepare the suspension $S_1$ by distillation of the aqueous solution or of the aqueous suspension $SA_2$. In other words, when an evaporator is used, it is the residence time of the ATBS (and/or the crystalline form of its sodium salt) between the inlet and the outlet of the device. This residence time depends on the quantity of water (and any organic solvents), ATBS.Na and sodium salt base present in the aqueous solution or the aqueous suspension $SA_2$. A person skilled in the art knows how to adapt this residence time in order to obtain ATBS.Na in the crystalline form depending on the quantity of the constituents of the aqueous solution or of the aqueous suspension $SA_2$.

The distillation can be carried out in a vertical or horizontal evaporator. Preferably, it is carried out in a vertical evaporator.

The aqueous solution or the aqueous suspension $SA_2$ can circulate co-current or counter-current to the vapours generated by the evaporation. Preferably, it circulates counter-current to the vapours in the distillation device. In other words, the aqueous solution or the aqueous suspension $SA_2$ is preferably introduced into the distillation device, advantageously an evaporator, co-current or counter-current to the distilled solvent.

The aqueous solution $SA_2$ or the aqueous suspension $SA_2$ may circulate in one or more evaporators in series before obtaining the suspension $S_1$. Preferably, it circulates in a single evaporator.

The pressure during distillation is advantageously between 1 and 700 mbar absolute (1 mbar = 100 Pa). It is preferably less than 700 mbar absolute, more preferably less than 600 mbar absolute, more preferably less than 500 mbar absolute, more preferably less than 400 mbar absolute, more preferably less than 300 mbar absolute, more preferably less than 200 mbar absolute, more preferably less than 100 mbar absolute and even more preferably less than 50 mbar absolute, and advantageously greater than 1 mbar absolute. The absolute pressure corresponds to the pressure relative to zero pressure (vacuum).

In general, the pressure during distillation is preferably comprised between 10 and 700 mbar, preferably between 20 and 700 mbar, preferably between 40 and 700 mbar more preferably between 40 and 600 mbar, more preferably between 40 and 500 mbar, more preferably between 40 and 400 mbar, more preferably between 40 and 300 mbar, more preferably between 40 and 200 mbar, more preferably between 40 and 100 mbar.

In one particular embodiment, step 2) comprises a step 2') (optional) for helping the solvent to evaporate. Step 2') consists in increasing the temperature of the aqueous solution or of the aqueous suspension $SA_2$, in other words the distillation according to step 2') is carried out under heat.

In some embodiments, in step 2), the aqueous solution or the aqueous suspension $SA_2$ is heated, advantageously at a temperature of between 5°C and 95°C, preferably between more than 10°C and 60°C, more preferably between more than 20°C and 40°C.

The heating during distillation may be carried out by various technologies. Examples include, but are not limited to, heating with steam, with hot water, with electricity, by steam compression, or indeed by using a heat pump. Thus, the distillation device may be of the double-walled type, with a hot heat-transfer fluid circulating between the two walls.

The aqueous solution or the aqueous suspension $SA_2$ is advantageously heated to a temperature between more than 5°C and 95°C, preferably between more than 10°C and 60°C, more preferably between more than 20°C and 40°C.

When the aqueous solution or the aqueous suspension $SA_2$ is heated, the temperature is advantageously greater than the temperature of step 1).

The temperature of the aqueous solution or of the aqueous suspension $SA_2$ advantageously increases at a ramp of between 0.1 and 10°C/hour, preferably between 0.2 and 9°C/hour, more preferably between 0.3 and 8°C/hour, and even more preferably between 0.5 and 5°C/hour.

In some embodiments, the temperature of the aqueous solution or of the aqueous suspension $SA_2$ advantageously increases at a ramp of between 10 and 150°C/hour, preferably between 30 and 110°C/hour, more preferably between 50 and 100°C/hour, and even more preferably between 60 and 90°C/hour.

The increase in temperature may not be constant throughout the whole process. For example, the aqueous solution or the aqueous suspension $SA_2$ may be heated by 5°C per hour for the first three hours, and then heated at a speed of 10°C per hour until the final temperature is reached.

According to another particular embodiment of the invention, step 2) comprises a step 2"), after step 2'), or instead of step 2'), that helps to increase the productivity and the profitability of the method of the invention by accelerating the crystallization of the ATBS into the crystalline form of its sodium salt. Step 2") consists in decreasing the temperature of

the aqueous solution or of the aqueous suspension $SA_2$ or the suspension S1.

The aqueous solution or the aqueous suspension $SA_2$ is advantageously cooled to a temperature of between 5 and less than 95°C, preferably between 10 and less than 60°C, more preferably between 20 and less than 40°C and even more preferably between 10 and 40°C.

Step 2) further comprises a cooling step.

The cooling step is advantageously carried out at a temperature of between 5°C and 95°C, preferably between more than 10°C and 60°C, more preferably between more than 10°C and 40°C.

The temperature of the cooling step is decreased at a ramp of between 0.1 and 8°C/hour, preferably between 0.2 and 8°C/hour, more preferably between 0.3 and 8°C/hour, and even more preferably between 0.5 and 5°C/hour.

In some embodiments, the temperature of the cooling step is advantageously inferior to the temperature of heating of step 2) and/or step 1).

The cooling step is carried out on the aqueous solution or the aqueous suspension $SA_2$ and/or the concentrated aqueous solution or the aqueous suspension $SA_2$ and/or the suspension $S_1$.

When the aqueous solution or the aqueous suspension $SA_2$ is cooled (step 2")), the temperature is advantageously less than the temperature of step 2) and optionally 2').

According to a preferred embodiment, the temperature of step 2") is the same or less than the temperature of step 1).

In some embodiments, no organic solvent or aqueous solution is added in step 2'' for obtaining the crystals of ATBS.Na.

The temperature of the aqueous solution or of the aqueous suspension $SA_2$ decreases at a ramp of between 0.1 and 8°C/hour, preferably between 0.2 and 8°C/hour, more preferably between 0.3 and 8°C/hour, and even more preferably between 0.5 and 5°C/hour.

The decrease in temperature may not be constant throughout the whole process. For example, the aqueous solution or the aqueous suspension $SA_2$ may be cooled by 5°C per hour for the first three hours, and then cooled at a speed of 8°C per hour until the final temperature is reached.

While the aqueous solution or the aqueous suspension $SA_2$ is being cooled, crystals of the sodium salt of ATBS form and a suspension $S_1$ is obtained.

In one particular embodiment, previously obtained crystals of sodium salt of ATBS may be added during this step in order to modify the formation of the suspension $S_1$, a process referred to as crystal seeding which makes it possible to better control the crystallization temperature, the particle size of the crystals, the particle size distribution, the purity of the end product and, possibly, the yield. The crystals of sodium salt of ATBS have advantageously an X-ray powder diffraction pattern comprising peaks at 11.7°; 12,2°; 13,2°; 13,5°; 15,6°; 16,8°; 17,8°; 18,5°; 19,1°; 20,6°; 21,4°; 23,3°; 25,1°; 25,8°; 26,9°; 29,1°; 29,5°; 31,0°; 33,0°; 33,6°; 34,4°; 35,2°; 35,9°; 37,1°; 38,4°; 39,6°; 41,1°; 42,9°; 45,1°; 46,0°; 47,2°; 47,6° 2-theta angles (+/- 0.1°).

According to one particular embodiment of the invention, the solvent distilled in step 2) may be partially or totally recycled in order to form the aqueous solution $SA_1$ of ATBS in step 1). In other words, the distilled solvent is advantageously at least partially recycled in the aqueous solution $SA_1$ of ATBS.

According to another particular embodiment of the invention, the distilled solvent may be partially or totally recycled, generally to wash the crystals of sodium salt of ATBS obtained after step 3) of solid-liquid separation, in an optional step 4), with or without a prior treatment step.

The obtained suspension $S_1$ advantageously comprises between 30 and 90% by weight of crystalline form of ATBS.Na, relative to the total weight of the suspension $S_1$, preferably between 50 and 90% by weight, more preferably between 30 and 80% by weight, and preferably between 50 and 60% by weight.

During step 2), the pH is advantageously greater than 10, preferably greater than 11, more preferably greater than 12, and the pH is even more preferably between 13 and 14.

**Step 3) of the method for producing the crystalline form of ATBS sodium salt:**

The crystals ATBS.Na contained in the suspension $S_1$ obtained at the end of step 2) are isolated in a solid-liquid separation step and are in the form of a composition $C_1$.

The solid-liquid separation step can be carried out using various technologies. Examples include, but are not limited to, the use of a centrifuge, a decanter, a filter press, an agitated filter, a belt filter, a disc filter or a rotary drum filter. The solid-liquid separation is preferably carried out using a centrifuge. The solid-liquid separation may also be carried out by gravitational settling.

Step 3) is advantageously carried out at a temperature of between -20 and 40°C, and preferably between -5 and 30°C.

After step 3) of solid-liquid separation, the crystals of sodium salt of ATBS are preferably not dried.

The isolated composition $C_1$ has a content of crystals of sodium salt of ATBS advantageously between 40 and

99%, preferably between 60 and 99% by weight, more preferably between 60 and 98% and even more preferably between 80 and 99% by weight relative to the weight of the composition $C_1$. The remainder of the composition $C_1$ may be water and/or solubilized sodium salt of ATBS, and possibly sodium salt base introduced in step 1).

At the end of this step 3), the crystals are characterized as being crystals of ATBS sodium salt (ATBS.Na).

In one particular embodiment, all or part of the liquid phase obtained following the solid-liquid separation is used in the aqueous solution $SA_1$ of step 1).

During step 4), the pH is advantageously controlled between 6 and 14, preferably between 8 and 14, more preferably between 10 and 14, even more preferably between 12 and 14, and even more preferably between 13 and 14.

**Step 4) of the method for producing the crystalline form of ATBS.Na:**

In an optional step 4), the composition $C_1$ containing the crystals of ATBS.Na obtained at the end of step 3) is washed using a washing solution.

The washing solution may be water, an aqueous solution of a sodium salt base (which may or may not be saturated), or a solution (which may or may not be saturated) of ATBS sodium salt (advantageously in the crystalline form of ATBS.Na), and it is preferably a saturated solution of ATBS.Na.

Examples of solutions of sodium salt base include a solution of sodium hydroxide, sodium carbonate, sodium bicarbonate or mixtures thereof.

The washing solution may comprise one or more organic solvents.

Advantageously, the washing solution does not comprise organic solvent.

As already indicated for step 1), the organic solvent is advantageously chosen from organic acids, amides, alcohols, ketones, ethers, esters, alkanes, halogenated hydrocarbon compounds, nitriles, or mixtures thereof. The organic solvent is preferably chosen from acrylonitrile, isopropanol, acetic acid or mixtures thereof. More preferably, the organic solvent is acrylonitrile.

In one particular embodiment, the composition $C_1$ obtained at the end of step 3) is washed by spraying washing solution over said composition $C_1$.

In one particular embodiment, the composition $C_1$ obtained at the end of step 3) is washed by placing the composition $C_1$ in suspension in the washing solution.

The weight ratio of the aqueous washing solution to the composition $C_1$ obtained at the end of step 3) is advantageously between 0.05:1 and 10:1 and more preferably between 0.1:1 and 5:1.

This washing step is advantageously carried out at a temperature of between -5 and 40°C, and preferably between 0 and 30°C. A person skilled in the art knows how to adjust the temperature so as not to solubilize the crystals of ATBS.Na.

The crystals of ATBS.Na obtained at the end of this optional step 4) can be isolated from the washing solution by a solid-liquid separation step, in the form of a composition $C_2$.

The solid-liquid separation step can be carried out using various technologies. Examples include, but are not limited to, the use of a vertical or horizontal centrifuge, a decanter, a filter press, a belt filter, a disc filter, a push filter or a rotary drum filter. The solid-liquid separation may also be carried out by gravitational settling.

In one particular embodiment, all or part of the recovered washing solution may be used again in step 4), with or without a prior treatment step.

In one particular embodiment, all or part of the recovered washing solution may be used in the aqueous solution $SA_1$ in step 1), with or without a prior treatment step.

The pH of the washing solution of step 5 is advantageously controlled between 6 and 14, and preferably between 8 and 14.

**Step 5) of the method for producing the crystalline form of ATBS.Na:**

In an optional step 5), the composition $C_1$ obtained at the end of step 3) or the composition $C_2$ obtained at the end of step 4) is dried.

The drying step can be carried out using various technologies. Examples include, but are not limited to, the use of all convection, conduction or radiation drying technologies (fluidized bed dryer, through-bed dryer, drying by conveyor belt, microwave, heated agitated filter, high-frequency radiation, infrared radiation, spraying).

The drying operation may be carried out at atmospheric pressure or else under vacuum.

The drying step may be carried out discontinuously (batch drying) or continuously.

**Other steps of the method for producing the crystalline form of ATBS.Na:**

During the production method, i.e., during steps 1) to 5), and regardless of the step, at least one polymerization

inhibitor may be introduced in order to prevent the possible polymerization of the ATBS or its salt. This polymerization inhibitor may be chosen, in a non-limiting manner, from hydroquinone, paramethoxyphenol, phenothiazine, 2,2,6,6-tetramethyl(piperidin-1-yl)oxyl, 4-hydroxy-2,2,6,6-tetramethyl(piperidin-1-yl)oxyl, phenylene diamine derivatives, or mixtures thereof.

The inhibitor is preferably paramethoxyphenol or 4-hydroxy-2,2,6,6-tetramethyl(piperidin-1-yl)oxyl.

The quantity of polymerization inhibitor that is introduced relative to the quantity of ATBS introduced in step 1) is advantageously between 0.001% and 5% by weight, and more preferably between 0.01% and 1% by weight.

The polymerization inhibitor may be introduced during any one or more of the steps of the method. An additional quantity of it is preferably introduced during step 1). More preferably, the polymerization inhibitor is part of the aqueous solution $SA_1$ introduced in step 1).

The production method (steps 1) to 5)) may be carried out continuously or discontinuously (batch production).

**Polymer**

In some embodiments, the novel crystalline form of ATBS.Na is used for producing polymers.

The polymer is obtained at least from ATBS, which is at least partially in the crystalline form of ATBS.Na having an X-ray powder diffraction pattern comprising peaks at 11,7°; 12,2°; 13,2°; 13,5°; 15,6°; 16,8°; 17,8°; 18,5°; 19,1°; 20,6°; 21,4°; 23,3°; 25,1°; 25,8°; 26,9°; 29,1°; 29,5°; 31,0°; 33,0°; 33,6°; 34,4°; 35,2°; 35,9°; 37,1°; 38,4°; 39,6°; 41,1°; 42,9°; 45,1°; 46,0°; 47,2°; 47,6° 2-theta angles (+/- 0.1°).

The polymer is obtained at least partially from the crystalline form of ATBS.Na, and advantageously from at least one other monomer chosen from: hydrophilic non-ionic monomers, hydrophilic anionic monomers (distinct from the crystalline form of ATBS.Na), hydrophilic cationic monomers, hydrophilic zwitterionic monomers and hydrophobic monomers.

It may therefore be a polymer of several distinct monomers or a homopolymer.

Advantageously, at least 10 mol%, preferably at least 30 mol%, more preferably at least 50 mol%, and even more preferably at least 70 mol% of the ATBS used to obtain the polymer is the crystalline form of ATBS.Na according to the invention. Even more preferably, 100 mol% of the ATBS is the crystalline form of ATBS.Na according to the invention.

The polymer advantageously comprises between 1 and 100 mol% of ATBS, preferably between 2 and 60 mol%, more preferably between 3 and 25 mol% and, advantageously, at least 10 mol%, preferably at least 30 mol%, more preferably at least 50 mol%, and even more preferably at least 70 mol% is the crystalline form of ATBS.Na. Even more preferably, 100 mol% of the ATBS that is used is the crystalline form of ATBS.Na according to the invention.

In one particular embodiment, the polymer advantageously comprises at least 10 mol% of ATBS, preferably at least 20 mol%, more preferably at least 30 mol%, more preferably at least 40 mol%, more preferably at least 50 mol%, more preferably at least 60 mol%, more preferably at least 70 mol%, more preferably at least 80 mol%, more preferably at least 90 mol% and, advantageously, at least 10 mol%, preferably at least 30 mol%, more preferably at least 50 mol%, and even more preferably at least 70 mol% is the crystalline form of ATBS.Na and, even more preferably, 100% of the ATBS that is used is the crystalline form of ATBS.Na according to the invention.

In one particular embodiment, the polymer is a homopolymer of ATBS and, advantageously, at least 10 mol%, preferably at least 30 mol%, more preferably at least 50 mol%, and even more preferably at least 70 mol% is in the crystalline form of ATBS.Na, and even more preferably 100% of the ATBS that is used is the crystalline form of ATBS.Na according to the invention.

In one particular embodiment, the polymer is a homopolymer of the crystalline form of ATBS.Na.

In one particular embodiment, the polymer is a polymer obtained from ATBS (at least 10 mol% of which is advantageously in the crystalline form of ATBS.Na ) and at least one non-ionic monomer.

**Composition of the polymer**

The polymer is obtained from the crystalline form of ATBS.Na, and advantageously from at least one other monomer which may be chosen from hydrophilic non-ionic monomers and/or hydrophilic anionic monomers and/or hydrophilic cationic monomers and/or hydrophilic zwitterionic monomers and/or hydrophobic monomers and mixtures thereof. It may be a polymer of several distinct monomers or a homopolymer.

Advantageously, the hydrophilic non-ionic monomer or monomers that can be used in the invention are chosen, in particular, from the group comprising water-soluble vinyl monomers such as acrylamide, methacrylamide, N-alkylacrylamides, N-alkylmethacrylamides, N,N-dialkylacrylamides (for example N,N-dimethylacrylamide or N,N-diethylacrylamide), N,N-dialkylmethacrylamides, alkoxylated esters of acrylic acid, alkoxylated esters of methacrylic acid, N-vinylpyrrolidone, N-vinyl caprolactam, N-vinylformamide (NVF), N-vinylacetamide, N-vinylimidazole, N-vinyl succinimide, acryloyl morpholine (ACMO), glycidyl methacrylate, glyceryl methacrylate, vinyl acetate, diacetone acrylamide, methacrylic anyhydride, acrylonitrile, maleic anydride, itaconamide, hydroxyalkyl (meth)acrylate, aminoalkyl (meth)acrylate, thioalkyl (meth)acrylate, hydroxy alkyl acrylates, hydroxy alkyl methacrylates, and mixtures thereof. Of these non-ionic monomers, the alkyl groups are advantageously $C_1$-$C_5$, and more advantageously $C_1$-$C_3$. They are preferably linear alkyls. Preferably, the hydrophilic non-ionic monomer is acrylamide.

The polymer advantageously comprises between 0 and 99 mol% of hydrophilic non-ionic monomer(s), preferably between 40 and 98 mol%, and more preferably between 75 and 97 mol%.

Advantageously, apart from the ATBS.Na in crystalline form, the hydrophilic anionic monomer or monomers which can be used in the invention can be chosen from a large group. These monomers may have vinyl functions (advantageously acrylic, maleic, fumaric, malonic, itaconic, or allylic), and contain a carboxylate, phosphonate, phosphate, sulphate or sulphonate group, or another anionically charged group. Examples of suitable monomers include acrylic acid; methacrylic acid; dimethylacrylic acid; itaconic acid; $C_1$-$C_3$ hemiesters of itaconic acid, crotonic acid; maleic acid; fumaric acid; acryloyl chloride, 3-acrylamido-3-methylbutanoic acid; maleic anhydride; strong acid monomers having, for example, a sulphonic acid or phosphonic acid function, such as vinylsulphonic acid, vinylphosphonic acid, allylsulphonic acid, methallylsulphonic acid, 2-methylidenepropane-1, 3-disulphonic acid, 2-sulphoethylmethacrylate, sulphopropylmethacrylate, sulphopropylacrylate, allylphosphonic acid, styrene sulphonic acid, ethylene glycol methacrylate phosphate, 2-acrylamido-2-methylpropanesulphonic acid (ATBS), 2-acrylamido-2-methylpropane disulphonic acid, 3-allyloxy-2-hydroxypropane sulfonic acid, diethylallylphosphonate; water-soluble salts of these monomers such as their alkali metal salts (distinct from the crystalline form of ATBS.Na), alkaline-earth metal salts or ammonium salts; and mixtures thereof. Preferably, the hydrophilic anionic monomer or monomers is acrylic acid and/or its salts.

The polymer advantageously comprises between 0 and 99 mol% of hydrophilic non-ionic monomer(s) (distinct from the crystalline form of ATBS.Na), preferably between 5 and 70 mol%, more preferably between 10 and 50 mol%. Above 5 mol%, these percentages also include the monomer in crystalline form of ATBS.Na according to the invention.

In one particular embodiment, the hydrophilic anionic monomer or monomers, apart from ATBS.Na in crystalline form, may be salified.

By salified, we mean the substitution of a proton of at least one acid function of the - $R^a$(=O)-OH type (with R representing P, S or C) of the anionic monomer by a metal or ammonium cation to form a salt of the -$R^a$(=O)-OX type (X being a metal cation or an organic cation). In other words, the non-salified form corresponds to the acid form of the monomer, for example $R^b$-C(=O)-OH in the case of the carboxylic acid function, while the salified form of the monomer corresponds to the $R^b$-C(=O)-$O^-$ $X^+$ form, $X^+$ corresponding to an alkaline cation or an organic cation. The salification of the acid functions of the branched water-soluble polymer can be partial or total.The salified form advantageously corresponds to the salts of alkali metals (Li, Na, K, etc.), alkaline-earth metals (Ca, Mg, etc.) or ammonium (for example the ammonium ion or a tertiary ammonium). The preferred salt is sodium salt.

The salification may take place before, during or after polymerization.

In one particular embodiment, the polymer advantageously comprises between 1 and 100 mol% of hydrophilic anionic monomer(s) in salified form, preferably between 50 and 100 mol%. These percentages include the monomer in crystalline form of ATBS.Na according to the invention.

Advantageously, the hydrophilic cationic monomer or monomers that can be used in the invention are chosen from monomers derived from vinyl-type units (advantageously acrylamide, acrylic, allylic or maleic), these monomers having a phosphonium or quaternary ammonium function. Mention may be made, in particular and in non-limiting manner, of diallyldialkyl ammonium salts such as diallyl dimethyl ammonium chloride (DADMAC); acidified or quaternized salts of dialkylaminoalkyl(meth)acrylamides, e.g. methacrylamido-propyl trimethyl ammonium chloride (MAPTAC), acrylamidopropyl trimethyl ammonium chloride (APTAC); acidified or quaternized salts of dialkylaminoalkyl acrylate, such as quaternized or salified dimethylaminoethyl acrylate (DMAEA); acidified or quaternized salts of dialkylaminoalkyl methacrylate, such as quaternized or salified dimethylaminoethyl methacrylate (DMAEMA); acidified or quaternized salts of N,N-dimethylallylamine; acidified or quaternized salts of diallylmethylamine; acidified or quaternized salts of diallylamine; polyvinylamine resulting from the hydrolysis (basic or acid) of an amide group -N(R2)-CO-R1 with R1 and R2 being, independently, a hydrogen atom or an alkylated chain of 1 to 6 carbons, for example polyvinylamine obtained from the hydrolysis of polyvinylformamide; polyvinylamine obtained by Hofmann degradation; and mixtures thereof. Advantageously, the alkyl groups are $C_1$-$C_7$, preferably $C_1$-$C_3$, and can be linear, cyclic, saturated or unsaturated chains. Preferably, quaternized dimethylaminoethyl acrylate.

A person skilled in the art knows how to prepare quaternized monomers, for example using a quaternizing agent of the R-X type, where R is an alkyl group and X is a halogen or sulfate.

The term "quaternizing agent" refers to a molecule capable of alkylating a tertiary amine.

The quaternizing agent may be chosen from dialkyl sulfates containing from 1 to 6 carbon atoms or alkyl halides containing from 1 to 6 carbon atoms. Preferably, the quaternizing agent is chosen from methyl chloride, benzyl chloride, dimethyl sulfate or diethyl sulfate.

In addition, the present invention also covers DADMAC, APTAC and MAPTAC monomers in which the counterion is a sulfate, fluoride, bromide or iodide instead of chloride.

The polymer advantageously comprises between 0 and 20 mol% hydrophilic cationic monomer(s), and preferably between 0 and 6 mol%.

Advantageously, the hydrophilic zwitterionic monomer or monomers may be a derivative of a vinyl-type unit (advantageously acrylamide, acrylic, allylic or maleic), this monomer having a quaternary amine or ammonium function and a carboxylic (or carboxylate), sulphonic (or sulphonate) or phosphoric (or phosphate) acid function. Mention may be made, in particular and in a non-limiting manner, of dimethylaminoethyl acrylate derivatives, such as 2-((2-(acryloyloxy) ethyl) dimethylammonio) dimethylammonio) ethane-1-sulphonate, 3-((2-(acryloyloxy)ethyl) dimethylammonio) propane-1-sulphonate, 4-((2-(acryloyloxy)ethyl) dimethylammonio) butane-1-sulphonate, [2-(acryloyloxy)ethyl] (dimethylammonio) acetate, dimethylaminoethyl methacrylate derivatives, such as 2-((2-(methacryloyloxy) ethyl) dimethylammonio) ethane-1-sulphonate, 3-((2-(methacryloyloxy) ethyl) dimethylammonio) propane-1-sulphonate, 4-((2-(methacryloyloxy) ethyl) dimethylammonio) butane-1-sulphonate, [2-(methacryloyloxy)ethyl] (dimethylammonio) acetate, dimethylamino propylacrylamide derivatives, such as 2-((3-acrylamidopropyl) dimethylammonio) ethane-1-sulphonate, 3-((3-acrylamidopropyl) dimethylammonio) propane-1-sulphonate, 4-((3-acrylamidopropyl) dimethylammonio) butane-1-sulphonate, [3-(acryloyloxy) propyl] (dimethylammonio) acetate, dimethylamino propyl methylacrylamide derivatives such as 2-((3-methacrylamidopropyl) dimethylammonio) ethane-1-sulphonate, 3-((3-methacrylamidopropyl) dimethylammonio) propane-1-sulphonate, 4-((3-methacrylamidopropyl) dimethylammonio) butane-1-sulphonate and [3-(methacryloyloxy)propyl] (dimethylammonio) acetate and mixtures thereof.

Other hydrophilic zwitterionic monomers can be used, in particular those described by the Applicant in document WO2021/123599.

The polymer advantageously comprises between 0 and 20 mol% hydrophilic zwitterionic monomer(s), more preferably between 0 and 10 mol%.

Hydrophobic monomers with a coefficient partition $K_{ow}$ greater than 1 can also be used in the preparation of the polymer according to the invention. They are preferably chosen from the following list: (meth)acrylic acid esters with a (i) $C_4$-$C_{30}$ alkyl, or (ii) arylalkyl ($C_4$-$C_{30}$ alkyl, $C_4$-$C_{30}$ aryl), or (iii) propoxylated, or (iv) ethoxylated, or (v) ethoxylated and propoxylated chain; alkyl aryl sulfonates ($C_4$-$C_{30}$ alkyl, $C_4$-$C_{30}$ aryl) ; mono- or disubstituted (meth)acrylamide amides bearing a (i) $C_4$-$C_{30}$ alkyl, or (ii) arylalkyl ($C_4$-$C_{30}$ alkyl, $C_4$-$C_{30}$ aryl), or (iii) propoxylated, or (iv) ethoxylated, or (v) ethoxylated and propoxylated chain; anionic or cationic monomer derivatives of (meth)acrylamide or (meth)acrylic acid bearing a hydrophobic chain; N-vinylpyridine and mixtures thereof. The hydrophobic monomers may comprise halogen atoms, for example chlorine.

Among these hydrophobic monomers:

- alkyl groups are preferably $C_4$-$C_{20}$, more preferably $C_4$-$C_8$. The $C_6$-$C_{20}$ alkyls are preferably linear, while the $C_4$-$C_5$ alkyls are preferably branched,
- arylalkyl groups are preferably $C_7$-$C_{25}$, more preferably $C_7$-$C_{15}$,
- the ethoxylated chains advantageously comprise between 1 and 200 $-CH_2$-$CH_2$-O-groups, preferably between 6 and 100, more preferably between 10 and 40,
- the propoxylated chains advantageously comprise between 1 and 50 $-CH_2$-$CH_2$-$CH_2$-O-groups, more preferably between 1 and 20.

Preferred hydrophobic monomers belonging to these classes are, for example:

- n-hexyl (meth)acrylate, n-octyl (meth)acrylate, octyl (meth)acrylamide, lauryl (meth)acrylate, lauryl (meth)acrylamide, myristyl (meth)acrylate, myristyl (meth)acrylamide, pentadecyl (meth)acrylate, pentadecyl (meth)acrylamide, cetyl (meth)acrylate, cetyl (meth)acrylamide, oleyl (meth)acrylate, oleyl (meth)acrylamide, erucyl (meth)acrylate, erucyl (meth)acrylamide, N-tert-Butyl (meth)acrylamide, 2-ethylhexyl acrylate, $C_4$-$C_{22}$ itaconic acid hemiesters, acidified or quaternized salts of $C_4$-$C_{22}$ dialkylaminoalkyl (meth)acrylate, acidified or quaternized salts of $C_4$-$C_{22}$ dialkylaminoalkyl (meth)acrylamides, vinylpyridine, acrylamido undecanoic acid, and mixtures thereof,
- cationic allyl derivatives of formula (I) or (II):

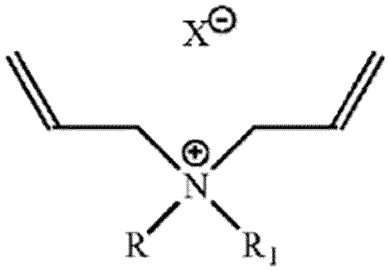

(I)

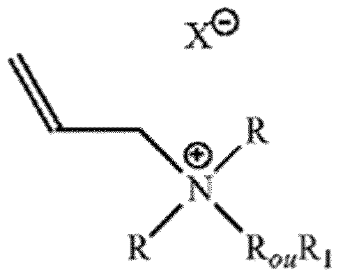

(II)

in which:

R: independently an alkyl chain containing 1 to 4 carbons;
$R_1$: an alkyl or arylalkyl chain containing 8 to 30 carbons;
X: a halide selected from the group consisting of bromides, chlorides, iodides, fluorides and any negatively charged counterion;
and, preferably, hydrophobic cationic derivatives of the (meth)acryloyl type corresponding to formula (III):

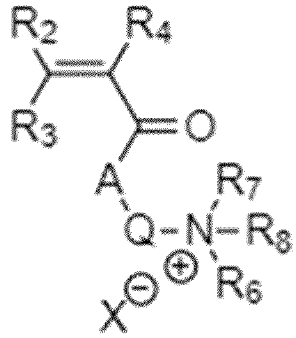

(III)

in which:

- A represents O or N-$R_5$ (preferably A represents N-$R_5$),
- $R_2$, $R_3$, $R_4$, $R_5$, $R_6$, $R_7$: independently hydrogen or an alkyl chain containing 1 to 4 carbons,
- Q: an alkyl chain containing 1 to 20 carbons,
- $R_8$: an alkyl or arylalkyl chain containing 8 to 30 carbons,
- X: a halide selected from the group consisting of bromides, chlorides, iodides, fluorides, and any negatively charged counterion.

When the polymer is water soluble, it advantageously comprises less than 5 mol% of hydrophobic monomers and the quantity thereof is adjusted in order for the polymer to remain soluble in water.

Monomers having a fluorescent function can also be used in the invention. A monomer with a fluorescent function may be detected by any suitable method, for example by fluorometry with a fixed wavelength fluorometer. Generally, the monomer with a fluorescent function is detected at the excitation and emission maxima, which can be determined using a scanning fluorometer.

Monomers with a fluorescent function are chosen, for example, from the following monomers: sodium or potassium styrene sulfonate, styrene sulfonic acid, vinylimidazole and its derivatives, 9-vinyl anthracene and its derivatives, N-9-xanthenylacrylamide and its derivatives, allyl dibenzosuberenol and its derivatives, chinconicin and its derivatives, quininone and its derivatives, cinchoninone and its derivatives, N,N-dimethyl-N-[3-[N'-(4-m'thoxy naphthalimide)]]propyl-N-(2-hydroxy-3-allyloxy)propyl ammonium hydroxide and mixtures thereof.

Other fluorescent compounds can be used when functionalized with an allyl, vinyl or acrylic double bond, such as pyranine and its derivatives, coumarin and its derivatives, quinolaxine and its derivatives, pinacyanol and its derivatives, xanthydrol and its derivatives, dabsyl and its derivatives, 3-hydroxy-2-methylene-3-(1-naphthyl)propionic acid and its derivatives, rhodamine and its derivatives, N-dibenzosuberylacrylamide and its derivatives, naphthalic derivatives,

fluorescein and its derivatives, pyrene and its derivatives, carbostyril and its derivatives, pyrazoline and its derivatives and mixtures thereof.

In a preferred mode, the polymer does not comprise a monomer with a fluorescent function.

In one particular embodiment, the polymer may comprise at least one cyclic monomer with a hydrolyzable function. Advantageously, the or the cyclic monomers with a hydrolyzable function are chosen from cyclic ketene acetals, thionolactones and mixtures thereof.

The cyclic ketene acetal is advantageously chosen from: 2-methylene-1,3-dioxepane (MDO), 5,6-benzo-2-methylene-1,3-dioxepane (BMDO), 2-methylene-4-phenyl-1,3-dioxolane (MPDL), 2-methylene-1,3,6-trioxocane (MTC), and mixtures thereof. Preferably, it is 2-methylene-1,3-dioxepane (MDO).

The thionolactone is advantageously chosen from: dibenzo[c,e]oxepine(7H)-5-thione (DOT), ε-thionocaprolactone, 3,3-dimethyl-2,3-dihydro-5H-benzo[e][1,4]dioxepine-5-thione (DBT) and mixtures thereof. Preferably, it is 3,3-dimethyl-2,3-dihydro-5Hbenzo[e][1,4]dioxepine-5-thione.

In one particular embodiment, the polymer may comprise at least one group with an LCST.

According to the general knowledge of a person skilled in the art, a group with an LCST corresponds to a group whose water solubility, for a given concentration, is modified above a certain temperature and as a function of salinity. It is a group with a heating transition temperature that defines its lack of affinity with the solvent medium. The lack of affinity with the solvent results in opacification or loss of transparency, which may be due to precipitation, aggregation, gelation or viscosification of the medium. The minimum transition temperature is known as the LCST (Lower Critical Solution Temperature). For each concentration a group with an LCST, a heating transition temperature is observed. It is higher than the LCST, which is the minimum point on the curve. Below this temperature, the polymer is soluble in water; above this temperature, the polymer loses its solubility in water.

In one particular embodiment, the polymer may comprise at least one group with an UCST.

According to the general knowledge of a person skilled in the art, a group with a UCST corresponds to a group whose water solubility, for a given concentration, is modified below a certain temperature and as a function of salinity. It is a group with a cooling transition temperature that defines its lack of affinity with the solvent medium. The lack of affinity with the solvent results in opacification or loss of transparency, which may be due to precipitation, aggregation, gelation or viscosification of the medium. The maximum transition temperature is known as the UCST (Upper Critical Solution Temperature). For each concentration a group with a UCST, a cooling transition temperature is observed. It is lower than the UCST, which is the maximum point on the curve. Above this temperature, the polymer is soluble in water; below this temperature, the polymer loses its solubility in water.

The quantities of the different monomer(s) will be adjusted by a person skilled in the art in order not to exceed 100 mol% when preparing the polymer according to the invention.

According to the invention, the polymer may have a linear, branched, cross-linked, star-shaped or comb-shaped structure. This structure can be obtained, according to the general knowledge of a person skilled in the art, for example by selecting the initiator, the transfer agent, the polymerization technique such as Reversible Addition Fragmentation chain Transfer (RAFT) polymerization, Nitroxide Mediated Polymerization (NMP) or Atom Transfer Radical Polymerization (ATRP), the incorporation of structural monomers, or the concentration.

The polymer may further by structured by a crosslinking agent. A structured polymer is a non-linear polymer that has side chains such that, when dissolved in water, the polymer has a high degree of entanglement leading to very high low-gradient viscosities.

The crosslinking agent is advantageously chosen from:

- structural agents, which can be chosen from the group comprising polyethylenically unsaturated monomers (having at least two unsaturated functions), such as vinyl functions, in particular allyl or acrylic functions, and we can cite for example methylene bis acrylamide (MBA), triallyamine, or tetraallylammonium chloride or 1,2 dihydroxyethylene bis-(N-acrylamide),
- monomers with at least two epoxy functions,
- monomers with at least one unsaturated and one epoxy function,
- macroinitiators such as polyperoxides, polyazoids and transfer agents such as polymercaptant polymers, and polyols,
- functionalized polysaccharides,
- water-soluble metal complexes composed of:

    * a metal with a valence greater than 3, such as, by way of example and non-limitation, aluminum, boron, zirconium or titanium, and
    * a ligand bearing a hydroxyl function.

The quantity of branching agent in the polymer is advantageously less than 40,000 ppm by weight relative to the

total weight of the monomers of the polymer, preferably less than 10,000 ppm by weight, and more preferably less than 5,000 ppm by weight.

In one particular embodiment, the quantity of branching agent is at least equal to 0.1 ppm by weight relative to the total weight of the monomers of the polymer, preferably at least 1 ppm by weight, more preferably at least 10 ppm by weight, more preferably at least 100 ppm by weight and even more preferably at least 1 000 ppm by weight.

When the polymer is water soluble and comprises a branching agent, the polymer remains soluble in water. A person skilled in the art knows how to adjust the quantity of branching agent and, possibly, the quantity of transfer agent needed to obtain this result.

In a preferred embodiment, the polymer is a water-soluble polymer comprising no branching agent.

In one particular embodiment, the polymer may comprise a transfer agent.

The transfer agent is advantageously chosen from methanol; isopropyl alcohol; sodium hypophosphite; calcium hypophosphite; magnesium hypophosphite; potassium hypophosphite; ammonium hypophosphite; formic acid; sodium formate; calcium formate; magnesium formate; potassium formate; ammonium formate; 2-mercaptoethanol; 3-mercaptopropanol; dithiopropylene glycol; thioglycerol; thioglycolic acid; thiohydracrylic acid; thiolactic acid; thiomalic acid; cysteine; aminoethanethiol; thioglycolates; allyl phosphites; allyl mercaptans, such as n-dodecyl mercaptan; sodium methallysulfonate; calcium methallysulfonate; magnesium methallysulfonate; potassium methallysulfonate; ammonium methallysulfonate; alkyl phosphites such as trialkyl ($C_{12}$-$C_{15}$) phosphites, di-oleyl-hydrogen phosphites, dibutyl phosphite; dialkyldithiophosphates such as dioctyl phosphonate; tertiary nonylmercaptan; 2-ethylhexyl thioglycolate; n-octyl mercaptan; n-dodecyl mercaptan; tertiary-dodecyl mercaptan; iso-octylthioglycolate; 2-ethylhexyl thioglycolate; 2-ethylhexyl mercaptoacetate; polythiols; and mixtures thereof. Preferably, the transfer agent is sodium hypophosphite or sodium formate.

The quantity of transfer agent in the polymer is advantageously between 0 and 100,000 ppm by weight relative to the total weight of the monomers of the polymer, preferably between 0 and 10,000 ppm by weight, more preferably between 0 and 1,000 ppm by weight, and even more preferably between 0 and 100 ppm by weight. When it is present, the transfer agent represents at least 0.1 ppm by weight relative to the total weight of the monomers of the polymer, and preferably at least 1 ppm by weight.

In one particular embodiment, the polymer comprises no transfer agent.

Generally speaking, the polymer does not require the development of any particular polymerization method. Indeed, it may be obtained using any of the polymerization techniques that are well known to a person skilled in the art. These include solution polymerization; gel polymerization; precipitation polymerization; emulsion polymerization (aqueous or inverse); suspension polymerization; reactive extrusion polymerization; water-in-water polymerization; or micellar polymerization.

The polymerization is generally radical polymerization, preferably by inverse emulsion polymerization or gel polymerization. Radical polymerization includes free radical polymerization using UV, azo, redox or thermal initiators, as well as controlled radical polymerization (CRP) or matrix polymerization techniques.

Controlled radical polymerization techniques include, but are not limited to, techniques such as Iodine Transfer Polymerization (ITP), Nitroxide Mediated Polymerization (NMP), Atom Transfer Radical Polymerization (ATRP), Reversible Addition Fragmentation chain Transfer (RAFT) Polymerization, which includes MADIX (MAcromolecular Design by Interchange of Xanthates) technology, various variations of Organometallic Mediated Radical Polymerization (OMRP), and OrganoHeteroatom-mediated Radical Polymerization (OHRP).

The polymer may be partially or totally post-hydrolyzed.

Post-hydrolysis is the hydrolysis reaction of the polymer after it has been formed by polymerization of the monomer(s). This step consists in reacting hydrolysable functional groups of monomers, advantageously non-ionic functional groups, more advantageously amide or ester functional groups, with a hydrolysis agent. This hydrolysis agent may, for example, be an enzyme, an ion-exchange resin, or a Brønsted acid metal (for example a hydrohalogenic acid) or a Brønsted base (for example an alkali hydroxide or an alkaline-earth hydroxide). Preferably, the hydrolysis agent is a Brønsted base. During this step of post-hydrolyzing the polymer, the number of carboxylic acid functions increases. Indeed, the reaction between the base and the amide or ester functions present in the polymer produces carboxylate groups.

The polymer may be in liquid, gel or solid form when its preparation includes a drying step such as spray drying, drum drying, radiation drying such as microwave drying, or drying in a fluidized bed.

The polymer advantageously has a molecular weight of at least 0.5 million g/mol, preferably between 0.5 and 40 million g/mol, more preferably between 5 and 30 million g/mol. Molecular weight is defined as weight-average molecular weight. The polymer may also have a molecular weight of between 5,000 and 100,000 g/mol or between 100,000 and 500,000 g/mol.

The molecular weight is determined by the intrinsic viscosity of the polymer. The intrinsic viscosity can be measured by methods known to a person skilled in the art and can be calculated from the reduced viscosity values for different polymer concentrations by a graphical method consisting in plotting the reduced viscosity values (y-axis) against

the concentration (x-axis) and extrapolating the curve to zero concentration. The intrinsic viscosity value is plotted on the y-axis or using the least-squares method. The molecular weight can then be determined using the Mark-Houwink equation:

$$[\eta] = K.M^{\alpha}$$

where $[\eta]$ represents the intrinsic viscosity of the polymer as determined by the solution viscosity method.
K represents an empirical constant.
M represents the molecular weight of the polymer.
$\alpha$ represents the Mark-Houwink coefficient.
K and $\alpha$ depend on the particular polymer-solvent system.

**Use of the polymer**

In some embodiments, the polymer may be used in: drilling or cementing wells; conformance, diversion; open, closed or semi-closed circuit water treatment; treatment of fermentation broth; sludge treatment; construction; paper or cardboard manufacture; batteries; wood treatment; treatment of hydraulic compositions (concrete, cement, mortar and aggregates); formulation of cosmetic products; formulation of detergents; textile manufacture; geothermal energy; manufacture of diapers; or agriculture.
In some embodiments, the polymer may be used as a coagulant, binding agent, absorbent agent, draining agent, filler retention agent, dehydrating agent, conditioning agent, stabilising agent, fixing agent, film-forming agent, sizing agent, superplasticizing agent, clay inhibitor or dispersant.
The invention and its advantages will be better understood in the light of the following figures and examples provided in order to illustrate the invention in a non-limiting manner.

**Description of the figures**

Figure 1 shows the proton NMR spectrum of ATBS obtained according to Example 1.

Figure 2 shows the proton NMR spectrum of ATBS sodium salt (ATBS.Na) crystals obtained according to Example 2a.

Figure 3 shows the X-ray diffraction pattern of the ATBS obtained according to example 1.

Figure 4 shows the X-ray diffraction pattern of the ATBS.Na crystals obtained according to example 2a.

Figure 5 shows the Fourier-transform infrared spectrum of the ATBS obtained in example 1.

Figure 6 shows the Fourier-transform infrared spectrum of the ATBS.Na crystals obtained in example 2a.

Figure 7 shows the thermogram of the ATBS obtained according to example 1.

Figure 8 shows the thermogram of the ATBS.Na crystals obtained according to example 2a.

Figure 9 shows the particle-size distribution of the ATBS obtained according to example 1.

Figure 10 shows the particle-size distribution of the ATBS.Na crystals obtained according to example 2a.

Figure 11 shows an optical microscope view of the ATBS obtained according to example 1.

Figure 12a shows an optical microscope view of the ATBS.Na crystals obtained according to example 2a.

Figure 12b shows an optical microscope view of the ATBS.Na crystals obtained according to example 2b.

Figure 12c shows an optical microscope view of the ATBS.Na crystals obtained according to example 2c.

Figure 13 shows the corrosion effect of the ATBS used, whether in acid form (example 1) or as crystals of sodium salt

(example 2a), on carbon steel plates.

Figure 14 shows a photo of the ATBS.Na product obtained according to Comparative example 2b (according to US6331647 (example 27)).

Figure 15 shows an optical microscope view of the ATBS.Na obtained according to Comparative example 2c (according to WO2013079507 (example 3).

Figure 16 shows an optical microscope view of the ATBS.Na obtained according to Comparative example 2d.

Figure 17 shows an optical microscope view of the ATBS.Na obtained according to Comparative example 2e.

**EXAMPLES**

AM = acrylamide
AA = acrylic acid
DMEA.MeCl = Dimethylaminoethylmethacrylate methyl chloride

**Example 1: Synthesis of the 2-acrylamido-2-methylpropanesulphonic acid (ATBS) ($A_H$)**

1522 g of acrylonitrile containing 0.4% by weight of water are added to a stirred, double-jacketed 2000 ml reactor, the mixture is stirred for 1 h and cooled by the double jacket of the reactor, which keeps the temperature of the sulphonating mixture at -20°C, and then 180 g of fuming sulphuric acid with a titre of 104% $H_2SO_4$ (18% oleum) are added.
97 g of isobutylene are added to the previous sulphonating mixture at a rate of 1.6 g/min.
The temperature of the mixture is controlled at 45°C when the isobutylene is added. The particles of the 2-acrylamido-2-methylpropanesulphonic acid precipitate out of the mixture and the solids content is approximately 20% by weight. The reaction mixture is filtered through a Büchner funnel and dried under vacuum at 50°C. The solid obtained is 2-acrylamido-2-methylpropanesulphonic acid (ATBS $A_H$) in the form of a very fine white powder.
Optical microscope observation (Figure 11) shows that the crystals of ATBS $A_H$ have a needle-shape morphology.

**Example 2a: Formation of the crystalline form of the 2-acrylamido-2-methylpropanesulphonic acid sodium salt (ATBS.Na $A_{Na}$2a) (according to the invention)**

439 g of a 22% (by weight in water) sodium hydroxide solution are added to a stirred, double-jacketed 1000 ml reactor. 452 g of ATBS $A_H$ of example 1 are added to the previous mixture.
The mixture is stirred for 30 min, at 10°C, to form an aqueous solution $SA_2$.
The aqueous solution $SA_2$ is heated to a temperature of 40°C under vacuum of 50 mbar, for 20 min, then the temperature is maintained for 30 min under a vacuum of 50 mbar and cooled to a temperature of 10°C. The cooling time between 40°C and 10°C is 6 h. A suspension $S_1$ of crystals of the ATBS.Na is obtained. The suspension $S_1$ is filtered on a Robatel vertical centrifuge. A solid of composition $C_1$ is obtained, containing 80% by weight of crystals of the ATBS.Na **$A_{Na}$2a.**
Optical microscope observation (Figure 12a) shows that the crystals **$A_{Na}$2a** have a columnar and platelet morphology.

**Example 2b: Formation of the crystalline form of the ATBS sodium salt (ATBS.Na $A_{Na}$2b)**

The crystals of ATBS.Na **$A_{Na}$2b** are prepared according to the procedure described in Example 2a except that $SA_2$ is distilled under 700 mbar.
Optical microscope observations (Figure 12b) show that the crystals **$A_{Na}$2b** obtained in these conditions are identical to the crystals of ATBS.Na **$A_{Na}$2a** prepared in example 2a.

**Example 2c: Formation of the crystalline form of the ATBS sodium salt (ATBS.Na $A_{Na}$2c)**

The crystals of ATBS.Na **$A_{Na}$2c** are prepared according to the procedure described in Example 2a except that the cooling time is reduced to 3h45.

Optical microscope observations (Figure 12c) show that show that the crystals obtained in these conditions are identical to the crystals of ATBS.Na **$A_{Na}$2a.**

**Comparative example 2a: Preparation of ATBS.Na under atmospheric pressure (1 bar) (CE- $A_{Na}$2a) (not obtained)**

The reaction is carried out according to the procedure described in Example 2a except that $SA_2$ is distilled under atmospheric pressure.
At the end of the cooling step, the aqueous solution $SA_2$ does not allow the formation of a suspension S1, and no filtration or centrifuging operation can be carried out to isolate crystals of ATBS sodium salt.

**Comparative example 2b: Preparation of ATBS sodium salt (CE- $A_{Na}$2b) (not obtained)**

The reaction was carried out according to the conditions described in example 27 of patent application US6331647.
124 g of sodium hydroxide and 0.13 g of hydroquinone monomethyl ether are added to a stirred, double-jacketed 5 000 ml reactor with 400 g of water. The medium is stirred until all the sodium hydroxide is dissolved.
632 g of ATBS **$A_H$** are added to the previous mixture. The mixture is stirred for 30 min, at 10°C, to form an aqueous solution of sodium salt of 2-acrylamido-2-methylpropanesulfonate.
The aqueous solution obtained is filtered in a 3 000 ml reactor equipped for distillation and containing an air purge tube. The content is heated and stirred while air is blown belon the surface at 0.5 cubic feet per h. The content is heated to 50°C while under a vacuum of 933 mbar (~700 millimeters of mercury). As the water is removed, a yellowish honeylike product forms. The product is then transfer to a Robatel vertical centrifuge, but no solid was recovered.
No optical microscope observation was possible as no solid was obtained (figure 14).

**Comparative example 2c: Preparation of ATBS sodium salt (CE-$A_{Na}$2c) (not obtained)**

The reaction was carried out according to the conditions described in example 3 of patent application WO2013079507.
100 g of an ATBS.Na solution (16.77% by weight) is obtained according to example 1 of WO2013079507.
50 g of solvent (mixture of acrylonitrile and methanol) are removed from the ATBS.Na solution at room temperature under reduced pressure (less than 700 mbar) while introducing air into the ATBS.Na solution. A solid of ATBS.Na **CE-$A_{Na}$2c** is formed and is filtered and washed with acrylonitrile/methanol and then dried at 50°C overnight.
Optical microscope observation (Figure 15) shows that the dried solid of ATBS.Na **CE-$A_{Na}$2c** does not correspond to the crystals of ATBS.Na according to the invention.

**Comparative example 2d: Preparation of ATBS.Na CE-$A_{Na}$2d (not according to the invention)**

The reaction is carried out according to the procedure described in Example 2a except that $SA_2$ is distilled under 720 mbar.
A solid of composition C1 is obtained, containing 80% by weight of crystals of the ATBS.Na **CE-$AN_a$2d**.
Optical microscope observation (Figure 16) shows that the crystals of ATBS.Na **CE-$A_{Na}$2d** does not correspond to the crystals of ATBS.Na according to the invention.

**Comparative example 2e: Preparation of ATBS.Na CE-$A_{Na}$2e (not according to the invention)**

The reaction is carried out according to the procedure described in Example 2a except that the cooling time is 3h20.
A solid of composition $C_1$ is obtained, containing 80% by weight of crystals of the ATBS.Na **CE-$A_{Na}$2e**.
Optical microscope observation (Figure 17) shows that the crystals of ATBS.Na **CE-$A_{Na}$2e** does not correspond to the crystals of ATBS.Na according to the invention.

**Example 3: NMR analysis of the products of ATBS $A_H$ and ATBS.Na $A_{Na}$2a from examples 1 and 2a**

ATBS **$A_H$**and ATBS.Na **$A_{Na}$2a** of are analysed by proton nuclear magnetic resonance (NMR).
The samples are dissolved in $D_2O$. The NMR machine is a Bruker model with a frequency of 400 MHz and is fitted with a 5 mm BBO BB-$^1$H.
The two proton spectra (Figures 1 and 2) are similar and the peak assignments are consistent with the molecular

structure of ATBS or the sodium salt.

**Example 4: Analysis by X-ray diffraction of products of ATBS $A_H$ and ATBS.Na $A_{Na}$2a from examples 1 and 2a**

ATBS $\mathbf{A_H}$ and crystals of ATBS.Na $\mathbf{A_{Na}}$**2a** are ground beforehand to form powders to be analysed by X-ray diffraction over an angular range of 10 to 90°. The equipment used is a Rigaku MiniFlex II diffractometer equipped with a copper source.

Crystals of ATBS.Na $\mathbf{A_{Na}}$**2a** (Figure 4) present an X-ray diffraction pattern with the following characteristic peaks: 11.7°; 12.2°; 13.2°; 13.5°; 15.6°; 16.8°; 17.8°; 18.5°; 19.1°; 20.6°; 21.4°; 23.3°; 25.1°; 25.8°; 26.9°; 29.1°; 29.5°; 31.0°; 33.0°; 33.6°; 34.4°; 35.2°; 35.9°; 37.1°; 38.4°; 39.6°; 41.1°; 42.9°; 45.1°; 46.0°; 47.2°; 47.6° 2-theta angles (+/- 0.1°).

The X-ray diffraction pattern of the ATBS $A_H$ (Figure 3) does not have the same peaks.

**Example 5: Fourier-transform infrared measurement of ATBS $A_H$ and ATBS.Na $A_{Na}$2a**

The equipment used for the Fourier transform infrared measurement is the Perkin Elmer Spectrum 100 fitted with a single reflection ATR polarization accessory, with an accuracy of 8 $cm^{-1}$.

ATBS $A_H$ and crystals of ATBS.Na $\mathbf{A_{Na}}$**2a** are sieved to 100 μm. The particles remaining on the sieve are dried and placed in an oven at 60°C for at least 4 h.

A few hundred milligram of solid are placed on the diamond of the ATR accessory and pressure is applied manually using the accessory.

The following bands (figure 6) are characteristic of the crystalline form of the ATBS.Na $\mathbf{A_{Na}}$**2a**:
3576$cm^{-1}$, 3485 $cm^{-1}$, 3310 $cm^{-1}$, 3079 $cm^{-1}$, 2975 $cm^{-1}$, 1658 $cm^{-1}$, 1629 $cm^{-1}$, 1543 $cm^{-1}$, 1403 $cm^{-1}$, 1321 $cm^{-1}$, 1301 $cm^{-1}$, 1205 $cm^{-1}$, 1187 $cm^{-1}$, 1163 $cm^{-1}$, 1046 $cm^{-1}$, 980 $cm^{-1}$, 629 $cm^{-1}$.

The infrared spectrum of ATBS $\mathbf{A_H}$ (figure 5) does not have the same peaks.

**Example 6: Differential Scanning Calorimetry (DSC) of ATBS $A_H$ and ATBS.Na $A_{Na}$2a products from Examples 1 and 2a**

The equipment used is a Mettler DSC 3.

ATBS $\mathbf{A_H}$ and crystals of ATBS.Na $\mathbf{A_{Na}}$**2a** are analysed with a heating ramp of 10°C/min under a flow of nitrogen. The initial temperature is 30°C, and the product is heated to 350°C.

The thermogram of ATBS $A_H$ (figure 7) shows a thermal effect at a temperature of 195.15°C, which is generally considered to be the melting/degradation point of ATBS, followed by two exothermic degradation phenomena at 212.8°C and 288.4°C.

The thermogram of the crystals of ATBS.Na $\mathbf{A_{Na}}$**2a** (figure 8) shows 4 thermal phenomena at 49.8°C; 144.8°C; 169.8°C; and 254.3°C.

**Example 7: Measurement of the minimum ignition energy (MIE) of ATBS $A_H$ and ATBS.Na $A_{Na}$2a products from Examples 1 and 2a**

The minimum ignition energy is measured in accordance with standard NF EN 13821.

The explosimeter is a vertical Hartmann tube. The dust dispersion system is of the mushroom type.

Total induction is less than 25 microhenry. Discharge voltage is between 5 kV and 15 kV. The electrodes are made of brass and spaced apart by a minimum distance of 6 mm.

Different energies and dispersed masses were tested and are summarized in the following tables.

It is clear that the crystalline form of ATBS.Na $\mathbf{A_{Na}}$**2a** presents a much lower risk of explosion than the needle form of ATBS $\mathbf{A_H}$.

Table 1: Determination of the solid MIE of ATBS $\mathbf{A_H}$

| Energy (mJ) | Weight of dispersed solids (g) | Number of dispersions | Inflammation?<br>Yes (Y)<br>No (N) | Flame | Pressure |
|---|---|---|---|---|---|
| 1000 | 0,5 | 2 | Y | Weak | Low |
| 500 | 0,5 | 3 | Y | Average | Average |

(continued)

| Energy (mJ) | Weight of dispersed solids (g) | Number of dispersions | Inflammation? Yes (Y) No (N) | Flame | Pressure |
|---|---|---|---|---|---|
| 300 | 0,5 | 3 | Y | Average | Average |
| 100 | 0,5 | 20 | N | | |
| 200 | 0,5 | 20 | N | | |
| 200 | 1 | 20 | N | | |
| 200 | 2 | 20 | N | | |
| 200 | 3 | 7 | Y | Average | Low |
| 100 | 3 | 20 | N | | |
| 100 | 5 | 20 | N | | |
| 100 | 7 | 20 | N | | |
| 100 | 10 | 20 | N | | |
| 100 | 1 | 20 | N | | |
| 100 | 2 | 20 | N | | |

Table 2: Determination of the solid MIE of ATBS.Na **$A_{Na}$2a**

| Energy (mJ) | Weight of dispersed solids (g) | Number of dispersions | Inflammation? Yes (Y) No (N)) | Flame | Pressure |
|---|---|---|---|---|---|
| 1000 | 0,5 | 20 | N | | |
| 1000 | 1 | 20 | N | | |
| 1000 | 2 | 20 | N | | |
| 1000 | 3 | 20 | N | | |
| 1000 | 3 | 20 | N | | |
| 1000 | 5 | 20 | N | | |
| 1000 | 7 | 20 | N | | |
| 1000 | 10 | 20 | N | | |
| 1000 | 15 | 20 | N | | |
| 1000 | 20 | 20 | N | | |
| High energy | 0,5 | 1 | Y | Weak | Low |

**Example 8: Particle-size measurement of ATBS $A_H$ and ATBS.Na $A_{Na}$2a products from Examples 1 and 2a**

The ATBS **$A_H$** and crystals of ATBS.Na **$A_{Na}$2a** are analysed by laser diffraction to determine their particle-size distribution.

The laser diffraction equipment used is a Cilas 1190.

The crystals of ATBS **$A_H$** have a $d_{50}$ value of approximately 40 µm and 90% of the particles are smaller than 100 µm (Figure 9).

The crystals of ATBS.Na **$A_{Na}$2a** have a $d_{50}$ value of approximately 600 µm and 90% of the particles are smaller than approximately 1500 µm (Figure 10). The crystals of ATBS.Na **$A_{Na}$2a** contain less than 10% of particles smaller than 325 µm.

**Example 9: Evaluation of the corrosivity of different forms of ATBS on carbon steel**

20 g of each ATBS $\mathbf{A_H}$ and $\mathbf{A_{Na}2a}$ and ATBS **CE-$\mathbf{A_{Na}}$2c to 2e** are deposited on two carbon steel plates measuring 20 x 50 mm. The coated plates are placed in an oven at 50°C for 15 days. At the same time, a control plate is left uncoated but placed under the same temperature conditions.

Photographs of the plates in this situation (Figure 13) show visually more pronounced corrosion on the plate that had been in contact with ATBS $\mathbf{A_H}$ compared to the crystals of $\mathbf{A_{Na}2a}$. Weighing the plates before and after the contact period confirms these observations.

Solid ATBS sodium salts **CE-$\mathbf{A_{Na}}$2c to 2e** have also been tested. The results are presented in Table 3.

Table 3: Corrosivity of different forms of ATBS on carbon steel

| | Initial weight (g) at t=0 | Final weight (g) t+15 days | % loss |
|---|---|---|---|
| Control plate | 9.5032 | 9.5020 | 0.012 |
| Plate +ATBS $\mathbf{A_H}$ | 9.3560 | 8.6582 | 7.46 |
| Plate +ATBS.Na $\mathbf{A_{Na}2a}$ | 9.4562 | 9.4302 | 0.275 |
| Plate +ATBS.Na **CE-$\mathbf{A_{Na}}$2c** | 9.4401 | 9.1224 | 3.36 |
| Plate +ATBS.Na **CE-$\mathbf{A_{Na}}$2d** | 9.3984 | 9,0130 | 4.10 |
| Plate +ATBS.Na **CE-$\mathbf{A_{Na}}$2e** | 9.5150 | 9,1103 | 4.25 |

**Example $10_a$: Preparation of a solution of ATBS.Na $A_{Na}2a$**

1000 g of the crystalline form of the ATBS.Na $\mathbf{A_{Na}2a}$ and 1000 g of water are introduced into a double-jacketed 2000 ml reactor fitted with a condenser, a pH meter and a stirrer.

The mixture has a pH greater than 12.

The mixture obtained is a solution of ATBS.Na at a concentration of 50% by weight in water.

**Example $10_b$: Preparation of a solution of ATBS.Na CE-$A_{Na}$2c**

The same protocol as in example $10_a$ is reproduced but using the crystal **CE-$\mathbf{A_{Na}}$2c**.

**Example $10_c$: Preparation of a solution of ATBS.Na CE-$A_{Na}$2d**

The same protocol as in example $10_a$ is reproduced but using the crystal **CE-$\mathbf{A_{Na}}$2d**.

**Example $10_d$: Preparation of a solution of ATBS.Na CE-$A_{Na}$2e**

The same protocol as in example $10_a$ is reproduced but using the ATBS.Na **CE-$\mathbf{A_{Na}}$2e**.

**Example 11: Preparation of a solution of ATBS.Na from the acid $A_H$**

800 g of ATBS $A_H$ and 650 g of water are introduced into a double-jacketed 2000 ml reactor fitted with a condenser, a pH meter and a stirrer. The mixture has a pH less than 1.

A 50% by weight sodium hydroxide in water solution is prepared in a dropping funnel. The caustic solution is added to the reaction mixture over 120 min. The temperature is controlled to less than 30°C.

The final pH of the solution is between 8 and 10.

310 g of 50% by weight sodium hydroxide in water solution are added.

The mixture obtained is a solution of ATBS.Na at a concentration of 50% by weight in water.

**Example 12: Influence of the form and structure of the ATBS on storage**

500 g of 50% (by weight in water) of the solutions of ATBS.Na prepared according to examples 10a-d and 11 were stored for 12 months in order to compare their stability over time by measuring and monitoring the appearance of homopolymers of ATBS.Na.

In parallel, the stability of the different ATBS (acid $\mathbf{A_H}$ or sodium salt $\mathbf{A_{Na}2a}$) in solid form was also assessed over

the same period of time.

In this case, every three months, 500 g of a solution of ATBS.Na was freshly prepared according to the preparation process described in example $10_a$ or 11 using the stored ATBS products of **$A_H$, $A_{Na}$2a** and **CE-$A_{Na}$2c to 2e.**

Stability of the solid products was also assessed by monitoring the amount of homopolymers of ATBS.Na present after that time.

The solutions were analysed by liquid-phase steric exclusion chromatography using an Agilent 1260 chromatograph equipped with Aquagel-OH 20, 30, 40 and 50 columns allowing analysis of anionic polymers up to 600,000 g/mol PEG equivalent.

The solutions of ATBS.Na were diluted to 2000 ppm (by weight in water) before being injected. The UV signal at 250 nm at the column outlet was integrated for the polymer peaks and are detailed in tables $4_a$ and $4_b$ below. The larger the signal area, the more polymer is present, and therefore the lower the stability of the product over time.

Table $4_a$: Stability of ATBS and ATBS sodium salt stored in solutions

| Sampling time after production | Solution according to example 10a stored (mV. s) | Solution according to example $10_b$ stored (mV.s) | Solution according to example $10_c$ stored (mV. s) | Solution from stored example $10_d$ (mV.s) | Solution according to example 11 stored (mV.s) |
|---|---|---|---|---|---|
| $T_0$ | 0 | 0 | 0 | 0 | 0 |
| $T_0$ + 3 months | 550 | 6157 | 6208 | 6389 | 6326 |
| $T_0$ + 6 months | 12536 | - | - | - | 311326 |
| $T_0$ + 9 months | 23564 | - | - | - | 562143 |
| $T_0$ + 12 months | 52364 | - | - | - | 1252230 |

Table $4_b$: Stability of ATBS and ATBS sodium salt stored in solid form

| Sampling time after production | Newly prepared solution from the stored crystals **$A_{Na}$2a** (mV. s) | Newly prepared solution from stored crystals **CE-$A_{Na}$2b** (mV.s) | Newly prepared solution from the stored crystals **CE-$A_{Na}$2c** (mV. s) | Newly prepared solution from the stored crystals **CE-$A_{Na}$2d** (mV. s) | Newly prepared solution from the stored crystals**$A_H$** (mV.s) |
|---|---|---|---|---|---|
| $T_0$ | 0 | 0 | 0 | 0 | 0 |
| $T_0$ + 3 months | 230 | 260 | 254 | 267 | 250 |
| $T_0$ + 6 months | 5263 | - | - | - | 7702 |
| $T_0$ + 9 months | 7593 | - | - | - | 10503 |
| $T_0$ + 12 months | 10235 | - | - | - | 256300 |

These results demonstrate that, whether stored in solid or as a solution, the crystalline form of ATBS.Na of the present invention presents an improved stability on storage over time.

**Example 13: Preparation of a polymer P1-$A_{Na}$2a of AM/ATBS (75/25 mol%)**

628.3 g of deionized water, 500 g of 50% (by weight in water) acrylamide solution, 16.2 g of urea and 268.8 g of crystals of ATBS.Na **$A_{Na}$2a** are added to a 2000 ml beaker.

The resulting solution is cooled to between 0 and 5°C and transferred to an adiabatic polymerization reactor, where it is bubbled with nitrogen for 30 min to remove all traces of dissolved oxygen.

The following are then added to the reactor:

- 0.75 g of 2,2'-azobisisobutyronitrile,
- 1.5 ml of a 5 g/L solution (in water) of 2,2'-azobis[2-(2-imidazolin-2-yl)propane] dihydrochloride,
- 1.5 ml of a 3 g/L solution (in water) of sodium hypophosphite,
- 2.25 ml of a 1 g/L solution (in water) of tert-butyl hydroperoxide,
- 2.25 ml of a 1 g/L solution (in water) of ammonium iron(II) sulphate (Mohr's salt).

After a few min, the nitrogen inlet is shut off and the reactor is closed. The polymerization reaction takes place for 1 to 5 h until a temperature peak is reached. The rubbery gel obtained is chopped into particles of between 1 and 6 mm in size.
The gel is then dried and ground to obtain polymer **P1-$A_{Na}$2a** in powder form.

**Example 14: Preparation of comparative polymers P1 of AM/ATBS (75/25 mol%)**

535.1 g of deionized water are added to a 2000 ml beaker, the solution is cooled to 5°C, and 243.0 g of crystals of ATBS **$A_H$** are added.
93.90 g of 50% (by weight in water) sodium hydroxide solution are prepared in a dropping funnel. As soon as a complete dissolution of the crystals is obtained, the caustic solution is added to the reaction mixture over 120 min. The temperature is controlled to be below 30°C. The final pH of the solution is between 8 and 10.
500 g of acrylamide in 50% solution (by weight in water) and 16.2 g of urea were added to complete the mixture.
The resulting solution is cooled to between 0 and 5°C and transferred to an adiabatic polymerization reactor, where it is bubbled with nitrogen for 30 min to remove all traces of dissolved oxygen.
The following are then added to the reactor:

- 0.75 g of 2,2'-azobisisobutyronitrile,
- 1.5 ml of a 5 g/L solution (in water) of 2,2'-azobis[2-(2-imidazolin-2-yl)propane] dihydrochloride,
- 1.5 ml of a 3 g/L solution (in water) of sodium hypophosphite,
- 2.25 ml of a 1 g/L solution (in water) of tert-butyl hydroperoxide,
- 2.25 ml of a 1 g/L solution (in water) of ammonium iron(II) sulphate (Mohr's salt).

After a few min, the nitrogen inlet is shut off and the reactor is closed. The polymerization reaction takes place for 1 to 5 h until a temperature peak is reached. The rubbery gel obtained is chopped into particles of between 1 and 6 mm in size.
The gel is then dried and ground to obtain the polymer **P1-$A_H$** in powder form.
The same protocol as in example 13 is reproduced but using the ATBS.Na **CE- $A_{Na}$2c** to produce polymer **P1-CE$A_{Na}$2c**.
The same protocol as in example 13 is reproduced but using the ATBS.Na **CE- $A_{Na}$2d** to produce. polymer **P1-CE$A_{Na}$2d**.
The same protocol as in example 13 is reproduced but using the ATBS.Na **CE- $A_{Na}$2e** to produce. polymer **P1-CE$A_{Na}$2e.**

**Example 15: Preparation of a homopolymer P2-$A_{Na}$2a of ATBS.Na $A_{Na}$2a**

562.1 g of deionized water and 389.4 g of crystals of the ATBS.Na **$A_{Na}$2a** are added to a 2000 ml beaker.
The resulting solution is cooled to between 5 and 10°C and transferred to an adiabatic polymerization reactor, where it is bubbled with nitrogen for 30 min to remove all traces of dissolved oxygen.
The following are then added to the reactor:

- 0.45 g of 2,2'-azobisisobutyronitrile,
- 1.5 ml of a 2.5 g/L solution (in water) of 2,2'-azobis[2-(2-imidazolin-2-yl)propane] dihydrochloride,
- 1.5 ml of a 1 g/L solution (in water) of sodium hypophosphite,
- 1.5 ml of a 1 g/L solution (in water) of tert-butyl hydroperoxide,
- 1.5 ml of a 1 g/L solution (in water) of ammonium iron(II) sulphate (Mohr's salt).

After a few min, the nitrogen inlet is shut off and the reactor is closed. The polymerization reaction takes place for 2 to 5 h until a temperature peak is reached. The rubbery gel obtained is chopped and dried to obtain a coarse powder which is itself ground and sieved to obtain the polymer **P2-$A_{Na}$2a** in powder form.

**Example 16: Preparation of comparative homopolymers P2 of ATBS**

427.5 g of deionized water are added to a 2000 ml beaker, the solution is cooled to 5°C, and 352.1 g of crystals of ATBS **$A_H$** are added under stirring.
136.1 g of 50% (by weight in water) sodium hydroxide solution are prepared in a dropping funnel. the caustic solution is added to the reaction mixture over 120 min. The temperature is controlled to be below 30°C. The final pH of the solution is between 8 and 10.
The resulting solution is cooled to between 5 and 10°C and transferred to an adiabatic polymerization reactor,

where it is bubbled with nitrogen for 30 min to remove all traces of dissolved oxygen.

The following are then added to the reactor:

- 0.45 g of 2,2'-azobisisobutyronitrile,
- 1.5 ml of a 2.5 g/L solution (in water) of 2,2'-azobis[2-(2-imidazolin-2-yl)propane] dihydrochloride,
- 1.5 ml of a 1 g/L solution (in water) of sodium hypophosphite,
- 1.5 ml of a 1 g/L solution (in water) of tert-butyl hydroperoxide,
- 1.5 ml of a 1 g/L solution (in water) of ammonium iron(II) sulphate (Mohr's salt).

After a few min, the nitrogen inlet is shut off and the reactor is closed. The polymerization reaction takes place for 2 to 5 h until a temperature peak is reached. The rubbery gel obtained is chopped and dried to obtain a coarse powder which is itself ground and sieved to obtain the polymer **P2-$A_H$** in powder form.

The same protocol as in example 15 is reproduced but using the ATBS.Na **CE-$A_{Na}$2c** to produce polymer **P2-$CEA_{Na}$2c.**

The same protocol as in example 15 is reproduced but using ATBS.Na **CE-$A_{Na}$2d** to produce. polymer **P2-$CEA_{Na}$2d**.

The same protocol as in example 15 is reproduced but using the ATBS.Na **CE-$A_{Na}$2e** to produce polymer **P2-$CEA_{Na}$2e**.

**Example 16a: Preparation of polymers P3 of AM/AA/ATBS (75/10/15 mol%)**

The experimental protocol of example 14 is reproduced except that the quantities of the different monomers are adjusted to reach the desired molar composition of acrylamide/acrylic acid/ATBS in the copolymer **P3-$A_{Na}$2a.**

Polymers **P3-$A_H$** is prepared using ATBS $A_H$.

Polymers **P3-$CEA_{Na}$2c** is prepared using ATBS.Na **CE-$A_{Na}$2c**.

Polymers **P3-$CEA_{Na}$2d** is prepared using ATBS.Na **CE-$A_{Na}$2d**.

Polymers **P3-$CEA_{Na}$2e** is prepared using ATBS.Na **CE-$A_{Na}$2e**.

**Example 16b: Preparation of polymers P4 of AM/DMEA.MeCl/ATBS (60/5/35 mol%)**

Polymers **P4-$A_{Na}$2a**, **P'4-$A_H$**, **P4-$CEA_{Na}$2c**, **P4-$CEA_{Na}$2d** and **P4-$CEA_{Na}$2e** are prepared according to the preparation process described in Example 16a.

**Example 16c: Preparation of polymers P5 of AM/DADMAC/ATBS (75/5/20 mol%)**

Polymers **P5-$A_{Na}$2a**, **P5-$A_H$**, **P5-$CEA_{Na}$2c**, **P5-$CEA_{Na}$2d** and **P5-$CEA_{Na}$2e** are prepared according to the preparation process described in Example 16a.

**Example 17: Viscosity measurement of polymers P1 to P5**

The reduced viscosity of the polymers prepared in examples 13 to 16 is measured at 25°C in a 0.5 M aqueous sodium chloride solution using a Brookfield LVT viscometer fitted with a UL adapter at 60 rpm.

Preparation of the polymer solutions:

500 mg of dried polymers are dissolved in a beaker containing 290 ml of deionized water at a stirring speed of 500 rpm.

9.75 g of sodium chloride are added to the prepared solutions.

The solutions are left to stir for 10 min at 700 rpm to dissolve the salt completely.

The prepared solutions are filtered through a 200 μm mesh.

16 ml of the prepared solutions is transferred to a cylindrical tube and used to carry out a viscosity measurement.

Table 5: Viscosity of polymers solutions of **P1**- **to P5-$A_{Na}$2a** according to the invention and comparative polymers **P1-$A_H$** and **P1 to P5-$CEA_{Na}$2c-e**; inv = invention and CE = comparative example

| Polymer | Viscosity (cps) |
|---|---|
| **P1-$A_{Na}$2a** (inv) | 7.2 |
| **P1-$A_H$** (CE) | 4.8 |

(continued)

| Polymer | Viscosity (cps) |
|---|---|
| **P1-CEA$_{Na}$2c** (CE) | 4.6 |
| **P1-CEA$_{Na}$2d** (CE) | 4.7 |
| **P1-CEA$_{Na}$2e** (CE) | 4.5 |
| **P2-A$_{Na}$2a** (inv) | 5.2 |
| **P2-A$_H$** (CE) | 3.5 |
| **P2-CEA$_{Na}$2c** (CE) | 3.3 |
| **P2-CEA$_{Na}$2d** (CE) | 3.1 |
| **P2-CEA$_{Na}$2e** (CE) | 3.2 |
| **P3-A$_{Na}$2a**(inv) | 6.1 |
| **P3-A$_H$** (CE) | 3.9 |
| **P3-CEAN$_a$2c** (CE) | 3.8 |
| **P3-CEA$_{Na}$2d** (CE) | 3.7 |
| **P3-CEA$_{Na}$2e** (CE) | 3.8 |
| **P4-A$_{Na}$2a** (inv) | 4.4 |
| **P4-A$_H$** (CE) | 2.9 |
| **P4-CEA$_{Na}$2c** (CE) | 3 |
| **P4-CEA$_{Na}$2d** (CE) | 2.7 |
| **P4-CEA$_{Na}$2e** (CE) | 2.8 |
| **P5-A$_{Na}$2a** (inv) | 6.9 |
| **P5-A$_H$** (CE) | 4.7 |
| **P5-CEA$_{Na}$2c** (CE) | 4.5 |
| **P5-CEA$_{Na}$2d** (CE) | 4.7 |
| **P5-CEA$_{Na}$2e** (CE) | 4.4 |

Polymers comprising the novel crystalline form of the ATBS.Na have a higher viscosity than polymers prepared from the conventional form of ATBS.Na, or those obtained from other crystalline forms.

## Claims

1. Crystaline form of 2-acrylamido-2-methylpropanesulphonic acid sodium salt ATBS.Na having an X-ray powder diffraction pattern comprising peaks at 11.7°; 12,2°; 13,2°; 13,5°; 15,6°; 16,8°; 17,8°; 18,5°; 19,1°; 20,6°; 21,4°; 23,3°; 25,1°; 25,8°; 26,9°; 29,1°; 29,5°; 31,0°; 33,0°; 33,6°; 34,4°; 35,2°; 35,9°; 37,1°; 38,4°; 39,6°; 41,1°; 42,9°; 45,1°; 46,0°; 47,2°; 47,6° 2-theta angles (+/- 0.1°).

2. Crystalline form of ATBS.Na according to claim 1, ***characterized* in that** it has a Fourier-transform infrared spectrum comprising peaks at 3576cm$^{-1}$, 3485 cm$^{-1}$, 3310 cm$^{-1}$, 3079 cm$^{-1}$, 2975 cm$^{-1}$, 1658 cm$^{-1}$, 1629 cm$^{-1}$, 1543 cm$^{-1}$, 1403 cm$^{-1}$, 1321 cm$^{-1}$, 1301 cm$^{-1}$, 1205 cm$^{-1}$, 1187 cm$^{-1}$, 1163 cm$^{-1}$, 1046 cm$^{-1}$, 980 cm$^{-1}$, 629 cm$^{-1}$ (+/- 8 cm-1).

3. Crystalline form of ATBS.Na according to claim 1 or 2, ***characterized* in that** it has a minimum ignition energy greater than 500 mJ.

4. Crystalline form of the sodium salt of ATBS.Na according to any one of claims 1 to 3, ***characterized* in that** it exhibits 4 thermal phenomena with the differential scanning calorimetry technique, at 49.8°C; 144.8°C; 169.8°C and 254.3°C (+/-10°C).

5. Method for producing the crystalline form of 2-acrylamido-2-methylpropanesulphonic acid of the sodium salt ATBS.Na according to any one of claims 1 to 4, comprising at least the following successive steps:

1) mixing of 2-acrylamido-2-methylpropanesulphonic acid with an aqueous solution $SA_1$ and at least one sodium salt base in order to form an aqueous solution or an aqueous suspension $SA_2$;
2) distillation, at a pressure of 700 mbar or less, of the aqueous solution or aqueous suspension $SA_2$ in order to form a suspension $S_1$ and cooling of the aqueous solution or aqueous suspension $SA_2$ or a suspension $S_1$ wherein the temperature of the cooling step is decreased at a ramp of between 0.1 and 8°C/hour;
3) solid-liquid separation of the suspension $S_1$ and isolation of the crystals of the suspension $S_1$ obtained at the end of step 2) in the form of a composition $C_1$.

6. Method according to claim 5, ***characterized in that*** the quantity of ATBS.Na in the aqueous solution or the aqueous suspension $SA_2$ is between 10 and 90% by weight relative to the total weight of the aqueous solution or of the aqueous suspension $SA_2$.

7. Method according to claim 5 or 6, ***characterized* in that** in step 2), the aqueous solution or the aqueous suspension $SA_2$ is heated, advantageously at a temperature of between 5°C and 95°C.

8. Method according to any one of claims 5 to 7, ***characterized* in that** the cooling step of step 2) is carried out at a temperature of between 5°C and 95°C.

9. Method according to any one of claims 5 to 8, ***characterized* in that** the suspension $S_1$ comprises between 30 and 90% by weight of crystalline form of ATBS.Na, relative to the total weight of the suspension $S_1$.

10. Method according to one of claims 5 to 9, ***characterized* in that** the sodium salt base is chosen from sodium hydroxide, sodium carbonate, sodium bicarbonate or mixtures thereof.

## Patentansprüche

1. Kristallines ATBS.Na, Natriumsalz der 2-Acrylamido-2-methylpropansulfonsäure, das ein Röntgenpulverdiffraktogramm mit Spitzen bei 11,7°; 12,2°; 13,2°; 13,5°; 15,6°; 16,8°; 17,8°; 18,5°; 19,1°; 20,6°; 21,4°; 23,3°; 25,1°; 25,8°; 26,9°; 29,1°; 29,5°; 31,0°; 33,0°; 33,6°; 34,4°; 35,2°; 35,9°; 37,1°; 38,4°; 39,6°; 41,1°; 42,9°; 45,1°; 46,0°; 47,2°; 47,6° 2-Theta-Winkel (+/-0,1°) aufweist.

2. ATBS.Na in kristalliner Form nach Anspruch *1, **dadurch gekennzeichnet,*** **dass** es ein Infrarotspektrum der Fourier-Transformation mit Spitzen bei 3576 $cm^{-1}$, 3485 $cm^{-1}$, 3310 $cm^{-1}$, 3079 $cm^{-1}$, 2975 $cm^{-1}$, 1658 $cm^{-1}$, 1629 $cm^{-1}$, 1543 $cm^{-1}$, 1403 $cm^{-1}$, 1321 $cm^{-1}$, 1301 $cm^{-1}$, 1205 $cm^{-1}$, 1187 $cm^{-1}$, 1163 $cm^{-1}$, 1046 $cm^{-1}$, 980 $cm^{-1}$, 629 $cm^{-1}$ (+/-8 $cm^{-1}$) aufweist.

3. ATBS.Na in kristalliner Form nach Anspruch 1 oder 2, ***dadurch gekennzeichnet,*** **dass** es eine Mindestzündenergie von mehr als 500 mJ aufweist.

4. Natriumsalz ATBS.Na in kristalliner Form nach einem der Ansprüche 1 bis 3, ***dadurch gekennzeichnet,*** **dass** es 4 thermische Phänomene mit dem Verfahren der dynamischen Differenzkalometrie bei 49,8 °C; 144,8 °C; 169,8 °C und 254,3 °C (+/-10 °C) aufweist.

5. Verfahren zur Herstellung der kristallinen 2-Acylamido-2-methylpropansulfonsäure des Natriumsalzes ATBS.Na nach einem der Ansprüche 1 bis 4, das mindestens folgende aufeinanderfolgende Schritte umfasst:

1) Mischen von 2-Acrylamido-2-methylpropansulfonsäure mit einer wässrigen Lösung $SA_1$ und mindestens einer Natriumsalzbase, um eine wässrige Lösung oder eine wässrige Suspension $SA_2$ zu bilden;
2) Destillation der wässrigen Lösung oder der wässrigen Suspension $SA_2$ bei einem Druck von 700 mbar oder weniger, um eine Suspension $S_1$ zu bilden, und Abkühlung der wässrigen Lösung oder der wässrigen Suspension $SA_2$ oder einer Suspension $S_1$, wobei die Temperatur des Abkühlungsschritts mit einer Rampe zwischen 0,1 und 8 °C/Stunde verringert wird;
3) Fest-Flüssig-Trennung der Suspension $S_1$ und Isolierung der Kristalle der am Ende von Schritt 2) erhaltenen Suspension $S_1$ in Form einer Zusammensetzung $C_1$.

6. Verfahren nach Anspruch 5, ***dadurch gekennzeichnet,*** **dass** die Menge an ATBS.Na in der wässrigen Lösung oder der wässrigen Suspension $SA_2$ zwischen 10 und 90 Gew.-% bezogen auf das Gesamtgewicht der wässrigen Lösung oder der wässrigen Suspension $SA_2$ beträgt.

7. Verfahren nach Anspruch 5 oder 6, ***dadurch gekennzeichnet,*** **dass** die wässrige Lösung oder die wässrige Suspension $SA_2$ in Schritt 2) erhitzt wird, zweckmäßigerweise auf eine Temperatur zwischen 5 °C und 95 °C.

8. Verfahren nach einem der Ansprüche 5 bis 7, ***dadurch gekennzeichnet,*** **dass** der Schritt der Abkühlung von Schritt 2) bei einer Temperatur zwischen 5 °C und 95 °C durchgeführt wird.

9. Verfahren nach einem der Ansprüche 5 bis 8, ***dadurch gekennzeichnet,*** **dass** die Suspension $S_1$ zwischen 30 und 90 Gew.-% der kristallinen Form von ATBS.Na, bezogen auf das Gesamtgewicht der Suspension $S_1$, enthält.

10. Verfahren nach einem der Ansprüche 5 bis 9, ***dadurch gekennzeichnet,*** **dass** die Natriumsalzbase aus Natriumhydroxid, Natriumcarbonat, Natriumbicarbonat oder deren Mischungen ausgewählt ist.

## Revendications

1. Forme cristalline du sel de sodium de l'acide 2-acrylamido-2-méthylpropane sulfonique ATBS.Na ayant un diagramme de diffraction des rayons X sur poudre comprenant des pics à 11,7° ; 12,2° ; 13,2° ; 13,5° ; 15,6° ; 16,8° ; 17,8° ; 18,5° ; 19,1° ; 20,6° ; 21,4° ; 23,3° ; 25,1° ; 25,8° ; 26,9° ; 29,1° ; 29,5° 31,0° 33,0° 33,6° 34,4° ; 35,2° 35,9° 37,1° 38,4° 39,6° ; 41,1° ; 42,9° ; 45,1° ; 46,0° ; 47,2° ; 47,6°, degrés 2-thêta (+/- 0,1°).

2. Forme cristalline d'ATBS.Na selon la revendication 1, ***caractérisée*** **en ce qu'**elle présente un spectre infra-rouge à transformée de Fourier comprenant des pics à $3576cm^{-1}$, $3485cm^{-1}$, $3310cm^{-1}$, $3079cm^{-1}$, $2975cm^{-1}$, $1658cm^{-1}$, $1629cm^{-1}$, $1543cm^{-1}$, $1403cm^{-1}$, $1321cm^{-1}$, $1301cm^{-1}$, $1205cm^{-1}$, $1187cm^{-1}$, $1163cm^{-1}$, $1046cm^{-1}$, $980cm^{-1}$, $629cm^{-1}$ (+/- $8cm^{-1}$).

3. Forme cristalline d'ATBS.Na selon la revendication 1 ou 2, ***caractérisée*** **en ce qu'**elle présente une énergie minimale d'inflammation supérieure à 500 mJ.

4. Forme cristalline du sel de sodium d'ATBS.Na selon l'une des revendications 1 à 3, ***caractérisée*** **en ce qu'**elle présente 4 phénomènes thermiques avec la technique de calorimétrie différentielle à balayage, à 49,8°C ; 144,8°C ; 169,8°C et 254,3°C (+/-10°C).

5. Procédé de fabrication de la forme cristalline du sel de sodium de l'acide 2-acrylamido-2-méthylpropane sulfonique ATBS.Na selon l'une des revendications 1 à 4, comprenant au moins les étapes successives suivantes ;

   1) mélange d'acide 2-acrylamido-2-méthylpropane sulfonique avec une solution aqueuse $SA_1$ et au moins une base de sel de sodium afin de former une solution aqueuse ou une suspension aqueuse $SA_2$ ;
   2) distillation, à une pression de 700 mbar ou moins, de la solution aqueuse ou de la suspension aqueuse $SA_2$ afin de former une suspension $S_1$ et refroidissement de la solution aqueuse ou suspension aqueuse $SA_2$ ou une suspension $S_1$ où la baisse de la température de l'étape de refroidissement est réalisée avec une rampe entre 0.1 et 8°C/heure ;
   3) séparation solide / liquide de la suspension $S_1$ et isolement des cristaux de la suspension $S_1$ obtenue à l'issue de l'étape 2) sous la forme d'une composition $C_1$.

6. Procédé selon la revendication 5, ***caractérisé*** **en ce que** la quantité d'ATBS.Na dans la solution aqueuse ou la suspension aqueuse $SA_2$ est entre 10% et 90% en poids par rapport au poids de la solution aqueuse ou de la suspension aqueuse $SA_2$.

7. Procédé selon la revendication 5 ou 6, ***caractérisé*** **en ce que**, dans l'étape 2), la solution aqueuse ou la suspension aqueuse $SA_2$ est chauffée, avantageusement à une température entre 5°C et 95°C.

8. Procédé selon l'une des revendications 5 à 7, ***caractérisé*** **en ce que** l'étape de refroidissement de l'étape 2) est réalisée à une température entre 5°C et 95°C.

**9.** Procédé selon l'une des revendications 5 à 8, ***caractérisé*** **en ce que** la suspension $S_1$ comprend entre 30 et 90% en poids de forme cristalline d'ATBS.Na, par rapport au poids total de la suspension $S_1$.

**10.** Procédé selon l'une des revendications 5 à 9, ***caractérisé*** **en ce que** la base de sel de sodium est choisie parmi l'hydroxyde de sodium, le carbonate de sodium, le bicarbonate de sodium ou un de leurs mélanges.

[Fig. 1]

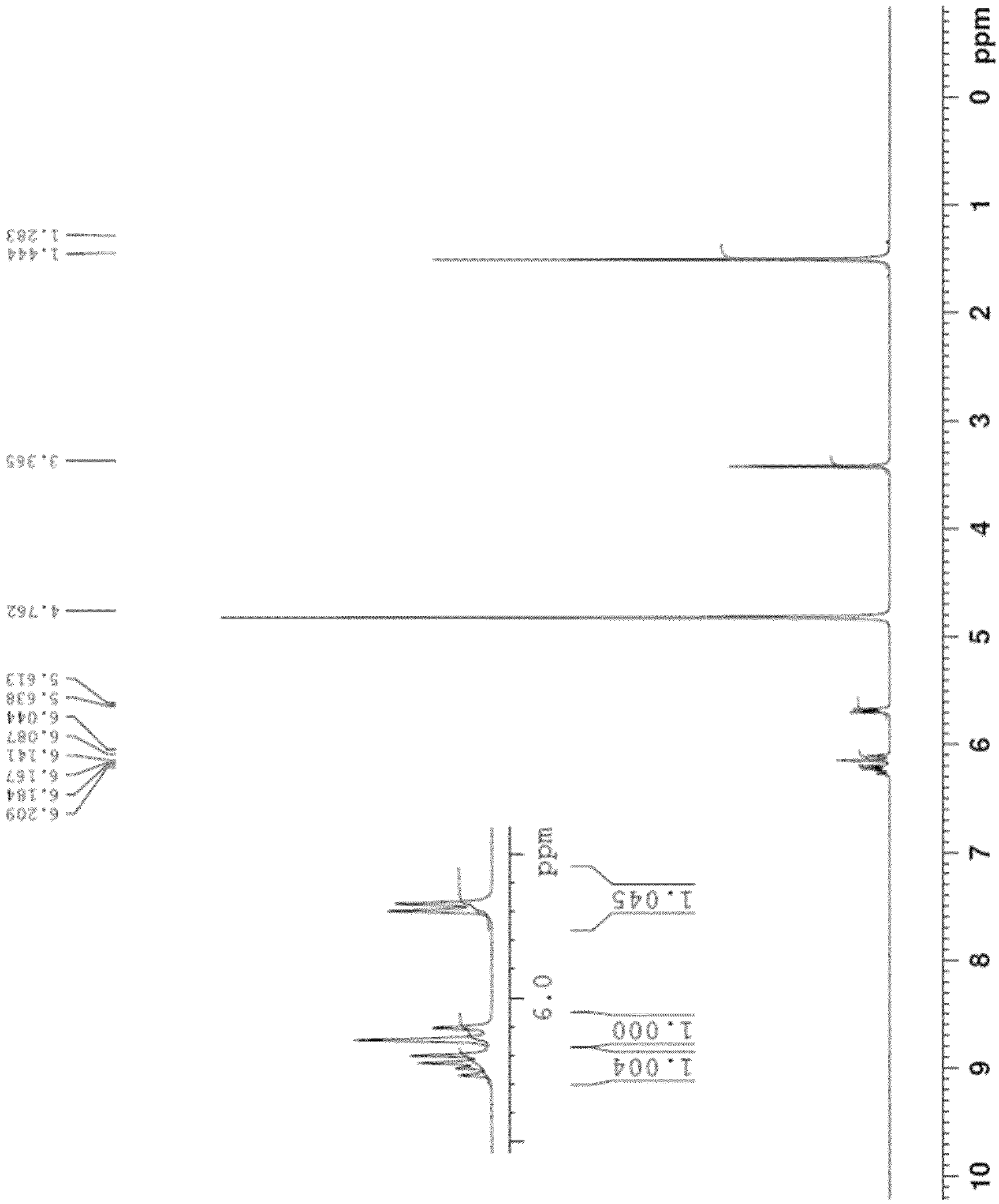
1.283
1.444
3.365
4.762
5.613
5.638
6.044
6.087
6.141
6.167
6.184
6.209
1.045
1.000
1.004
6.0
ppm
0 1 2 3 4 5 6 7 8 9 10
ppm

[Fig. 2]

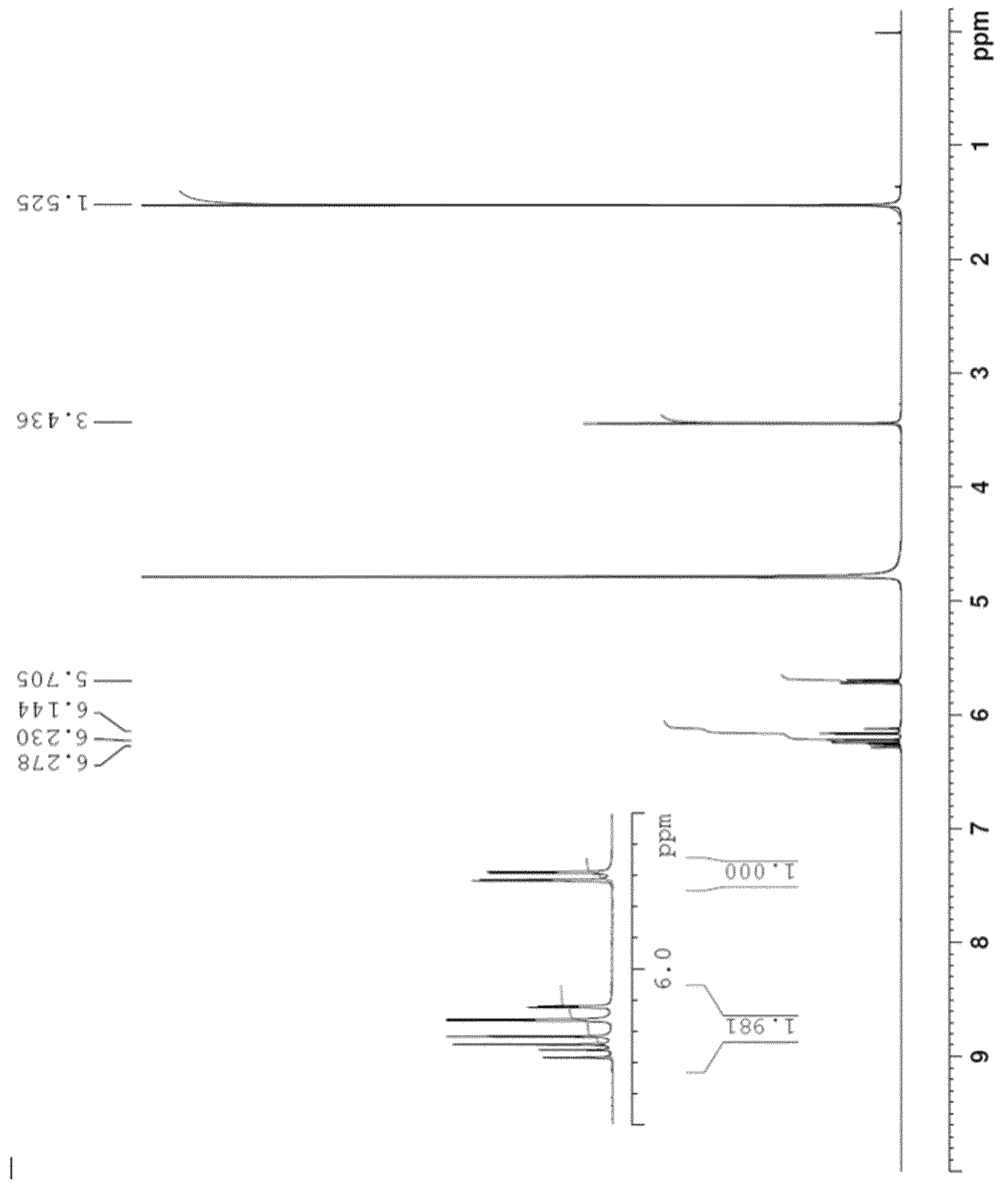

[Fig. 3]

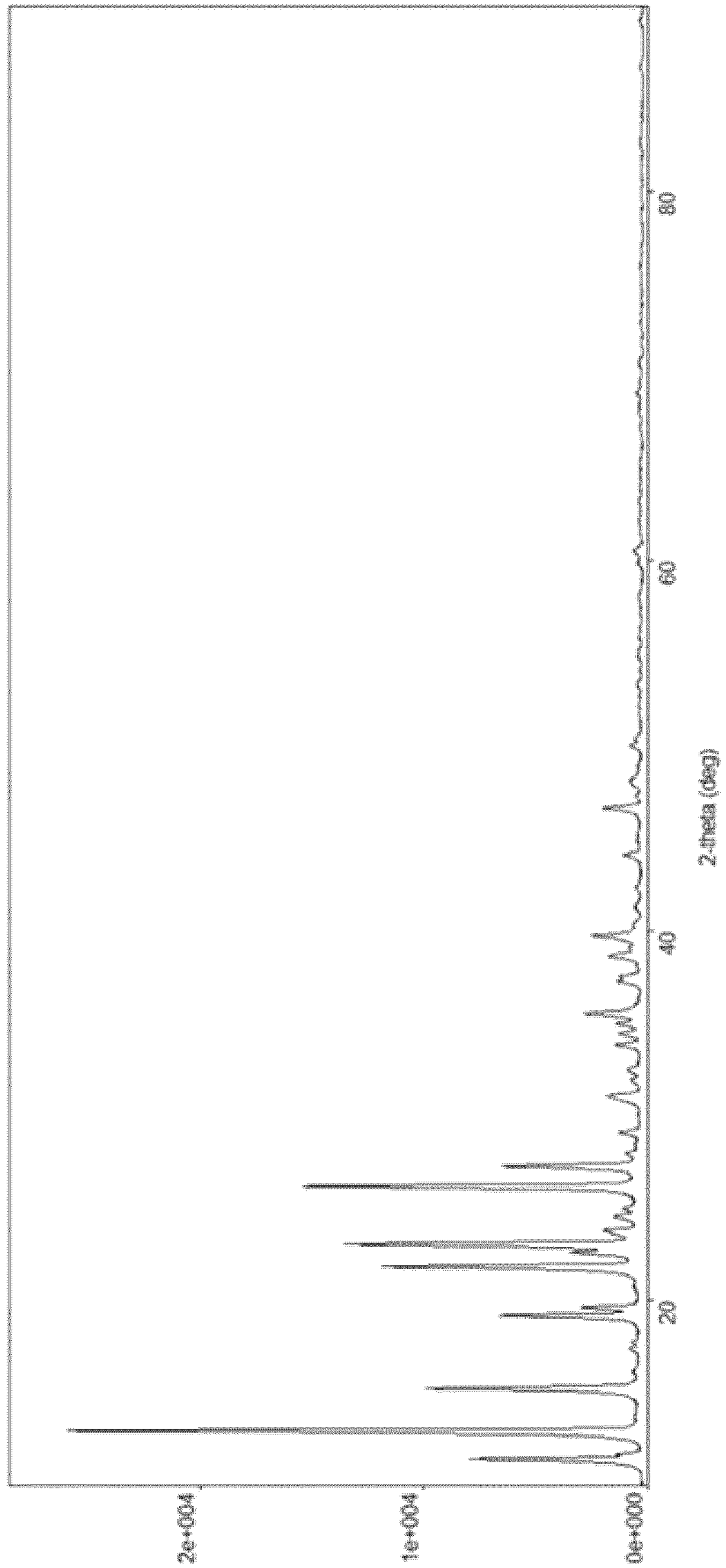

[Fig. 4]

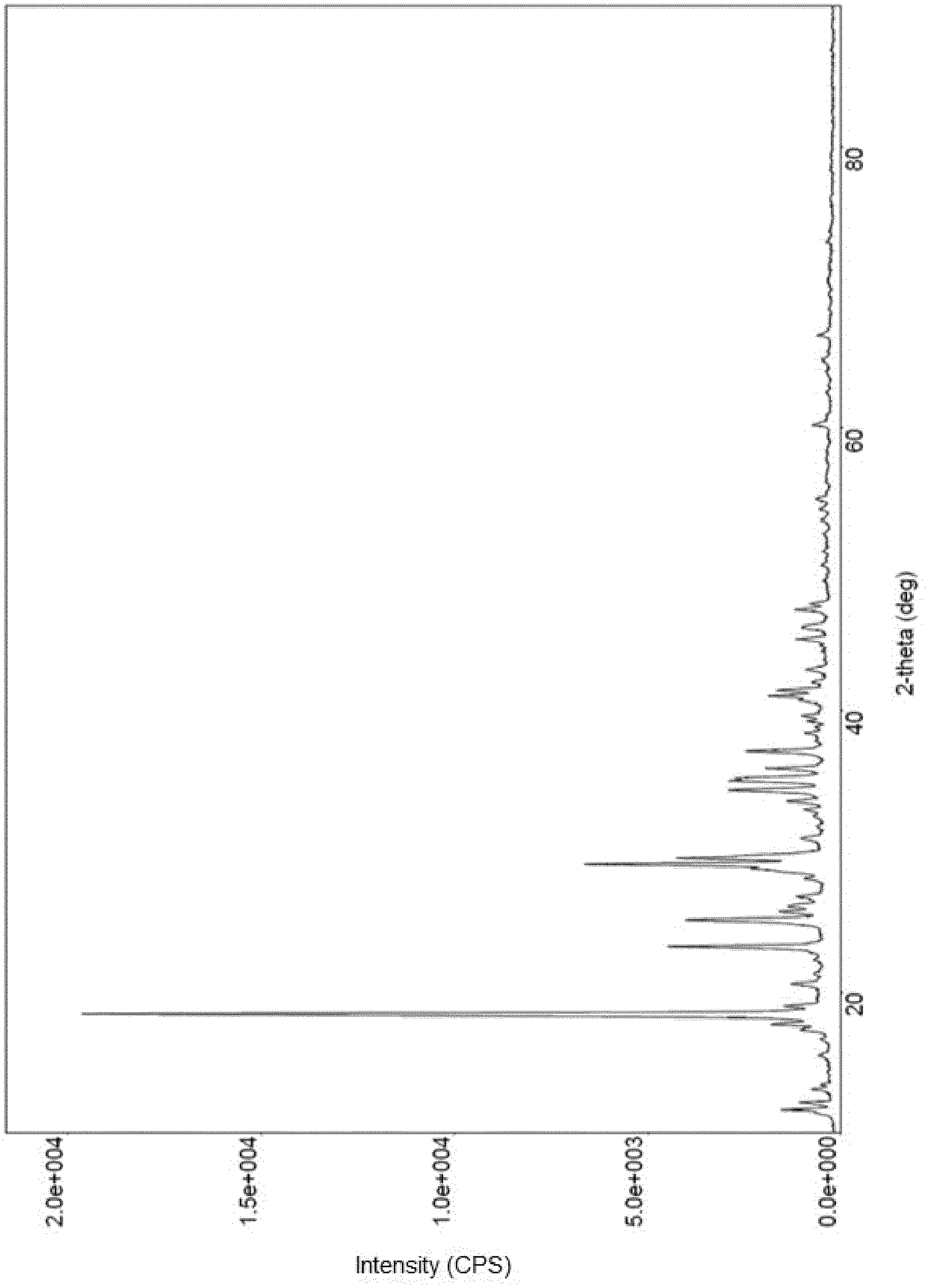
Intensity (CPS)
2.0e+004
1.5e+004
1.0e+004
5.0e+003
0.0e+000
2-theta (deg)
20
40
60
80

[Fig. 5]

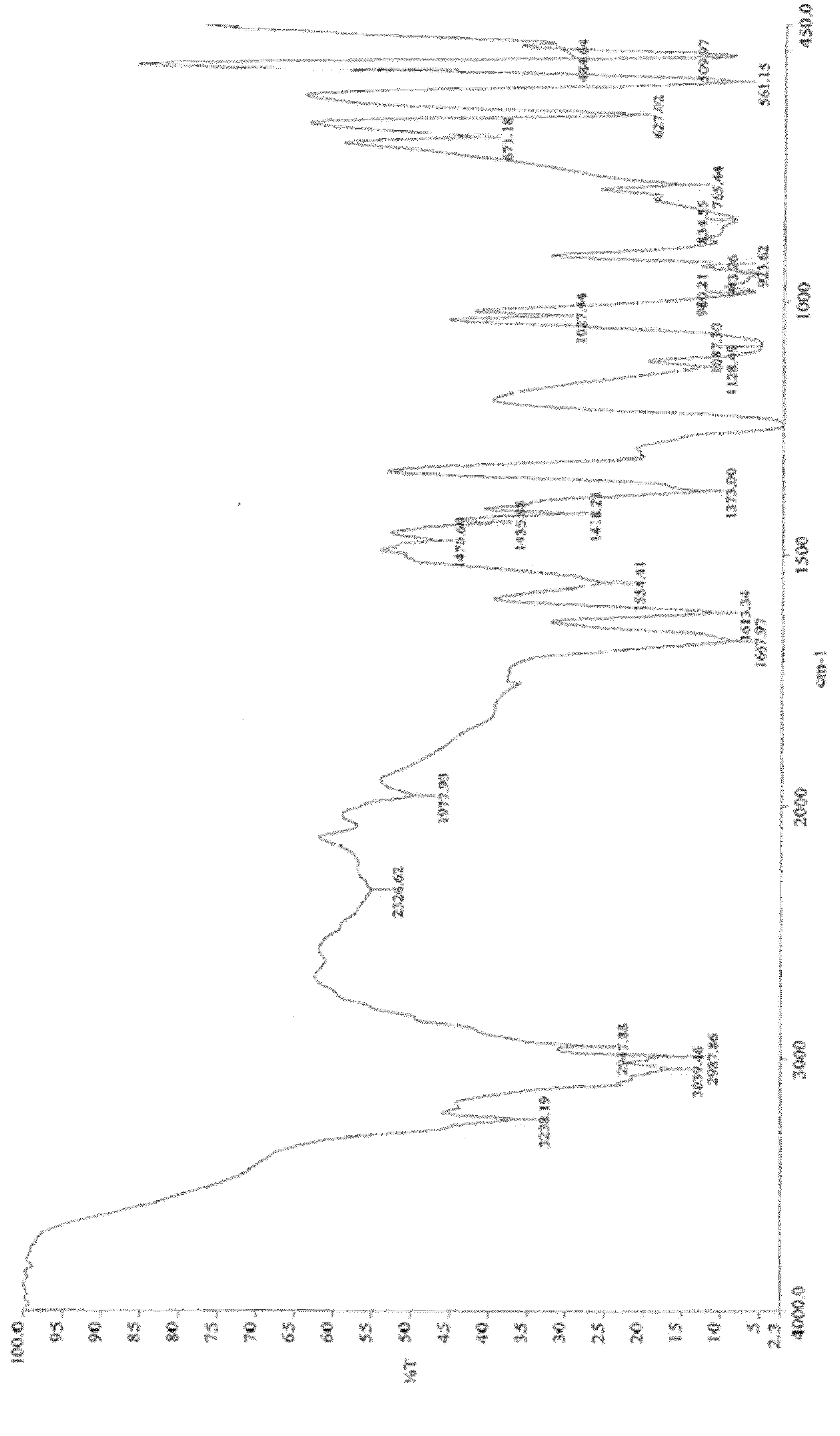
%T
cm-1
100.0
2.3
4000.0
3000
2000
1500
1000
450.0
3238.19
3039.46
2987.86
2947.88
2326.62
1977.93
1657.97
1613.34
1554.41
1470.60
1435.88
1418.23
1373.00
1128.49
1087.30
1027.44
980.21
923.62
834.55
765.44
671.18
627.02
561.15
509.97
484.64

[Fig. 6]

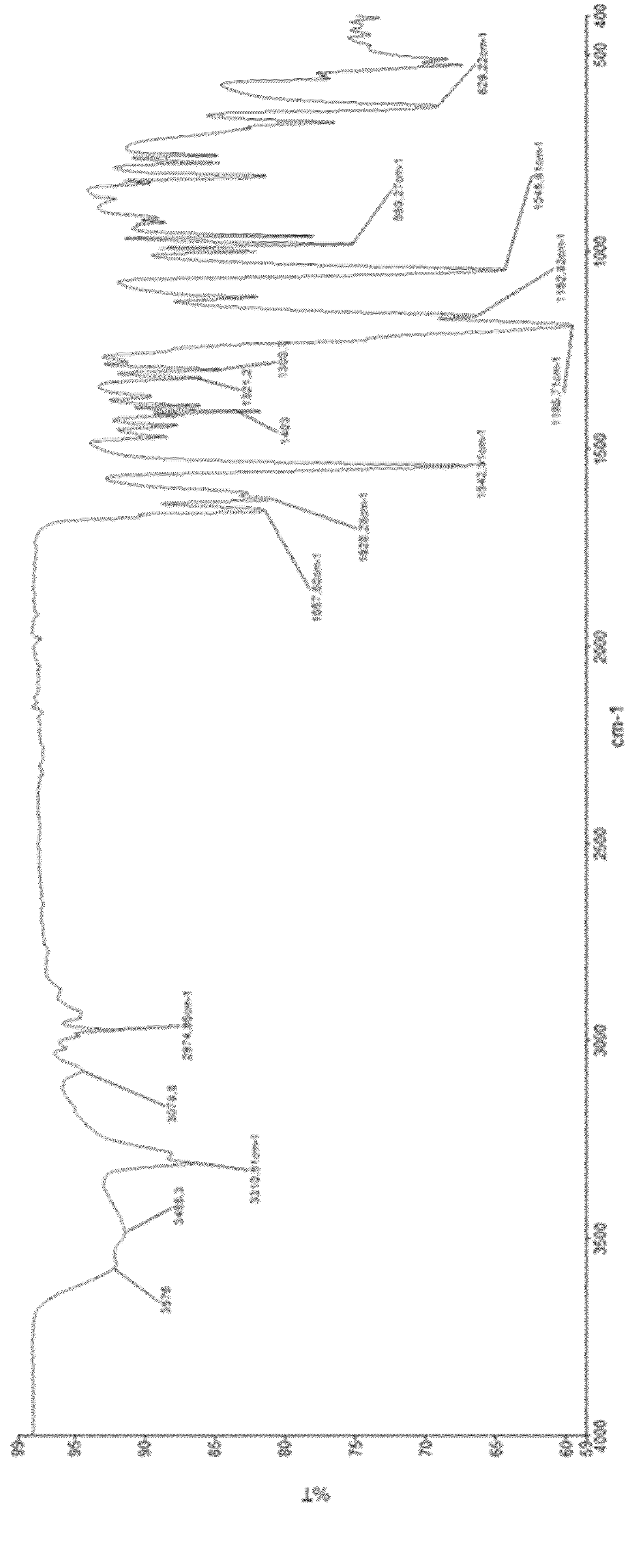
%T
99
95
90
85
80
75
70
65
60
59
4000
3500
3000
2500
2000
1500
1000
500
400
cm-1

[Fig. 7]

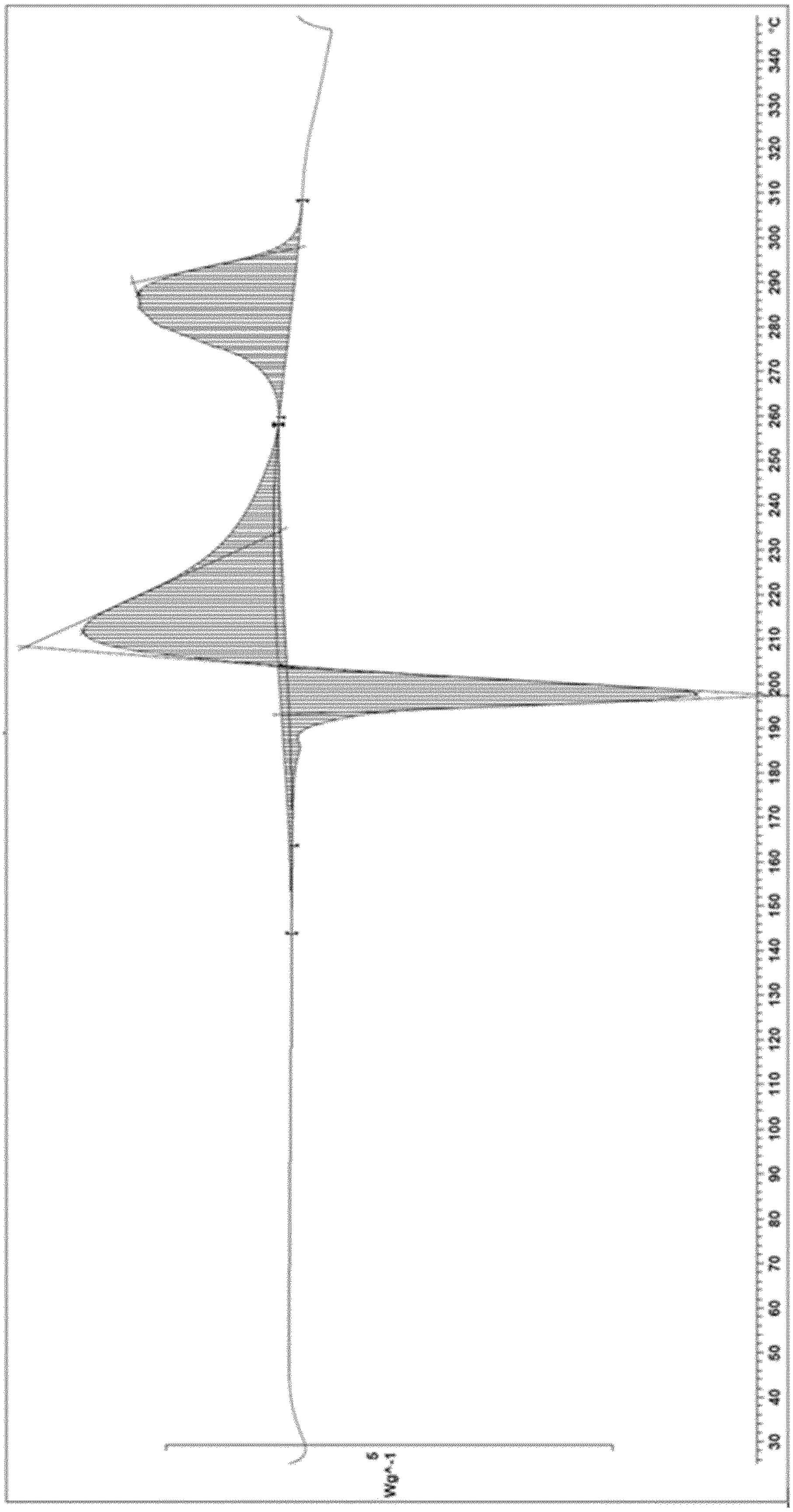
°C
30
40
50
60
70
80
90
100
110
120
130
140
150
160
170
180
190
200
210
220
230
240
250
260
270
280
290
300
310
320
330
340
5
Wg^-1

[Fig. 8]

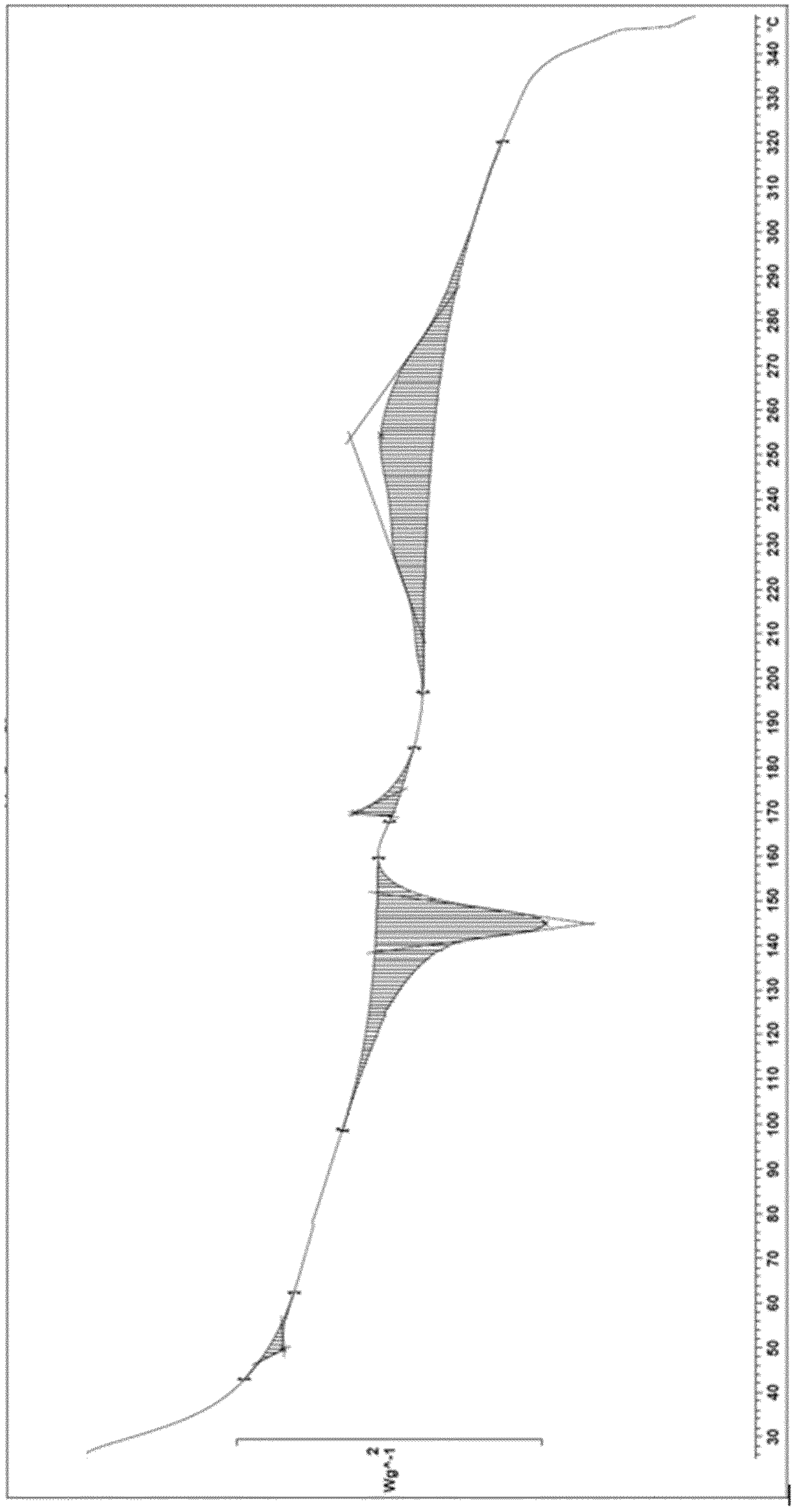
°C
30
40
50
60
70
80
90
100
110
120
130
140
150
160
170
180
190
200
210
220
230
240
250
260
270
280
290
300
310
320
330
340
2
Wg^-1

[Fig. 9]

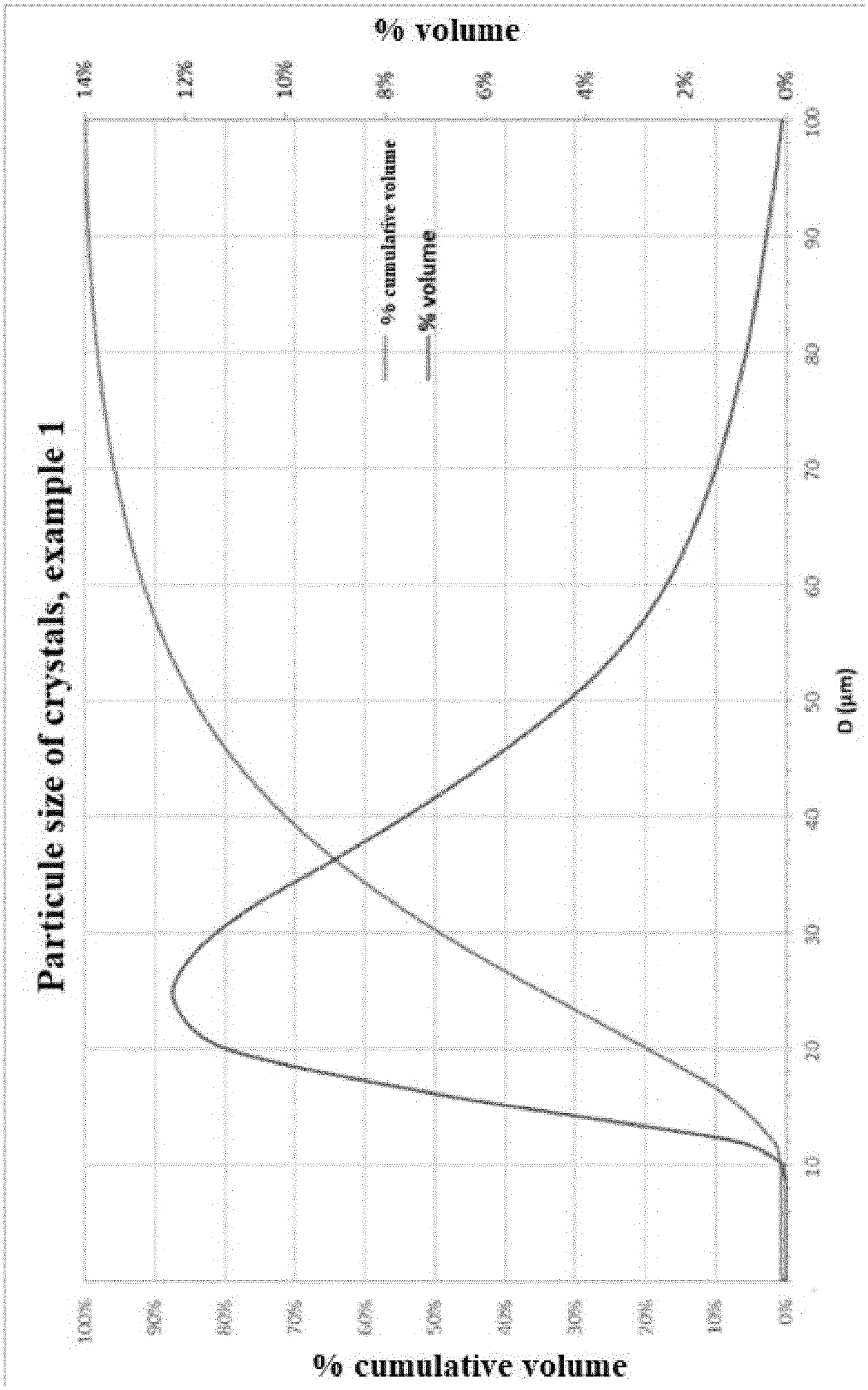
Particule size of crystals, example 1
% volume
14%
12%
10%
8%
6%
4%
2%
0%
% cumulative volume
% volume
D (µm)
0
10
20
30
40
50
60
70
80
90
100
100%
90%
80%
70%
60%
50%
40%
30%
20%
10%
0%
% cumulative volume

[Fig. 10]

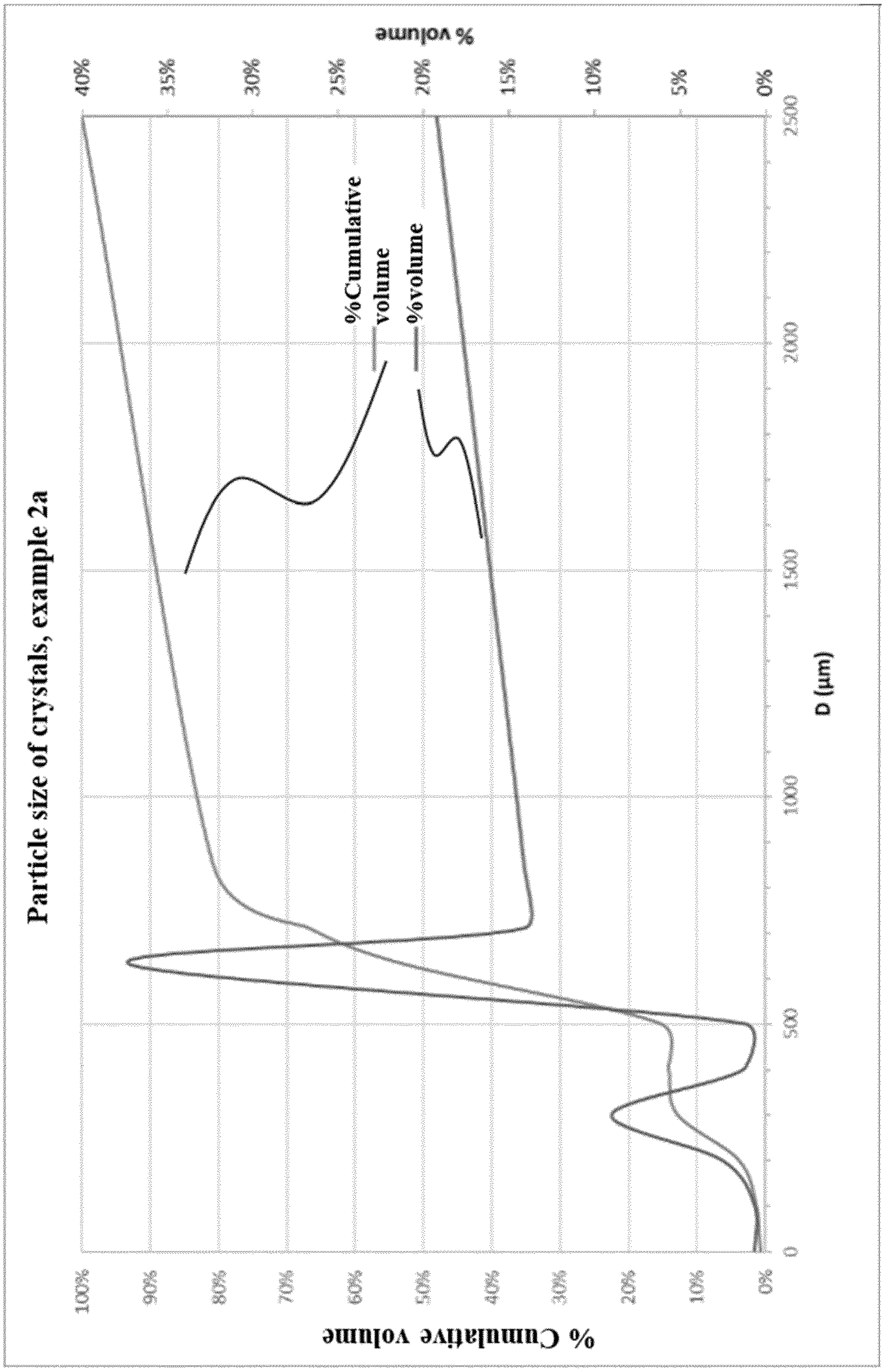

Particle size of crystals, example 2a
% Cumulative volume
% volume
D (µm)
%Cumulative volume
%volume
0%
10%
20%
30%
40%
50%
60%
70%
80%
90%
100%
5%
15%
25%
35%
0
500
1000
1500
2000
2500

[Fig. 11]

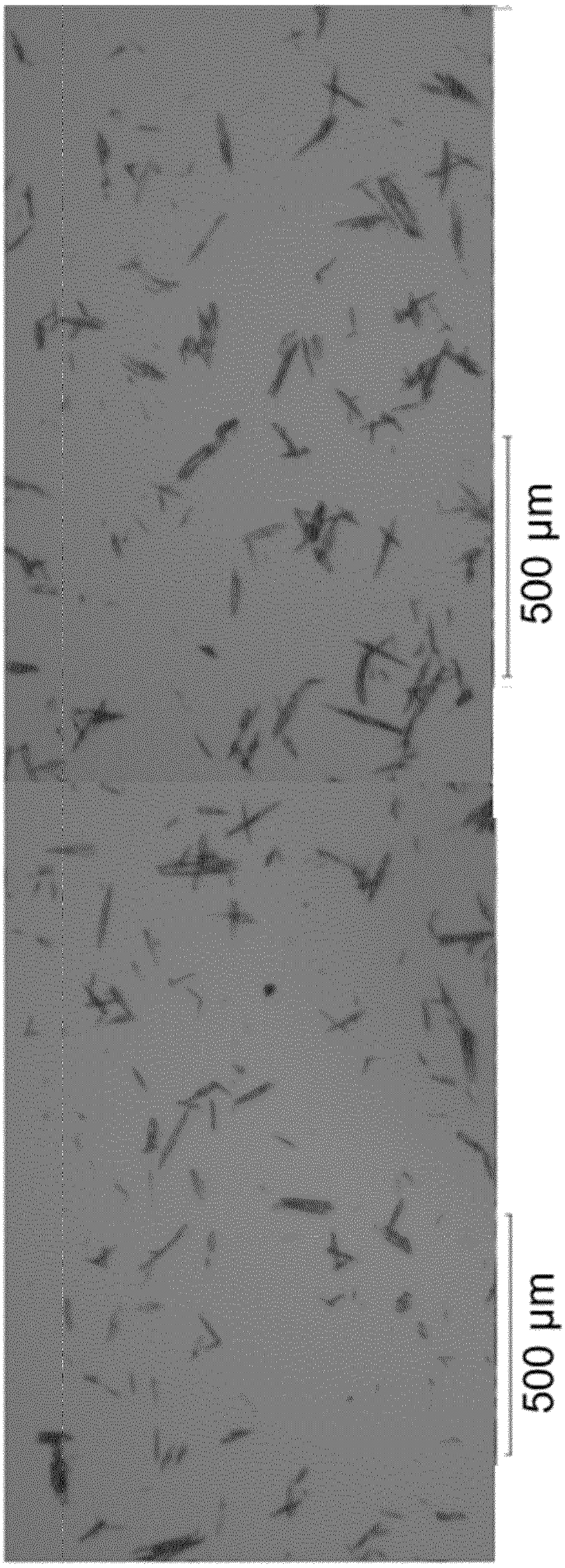

[Fig. 12a]

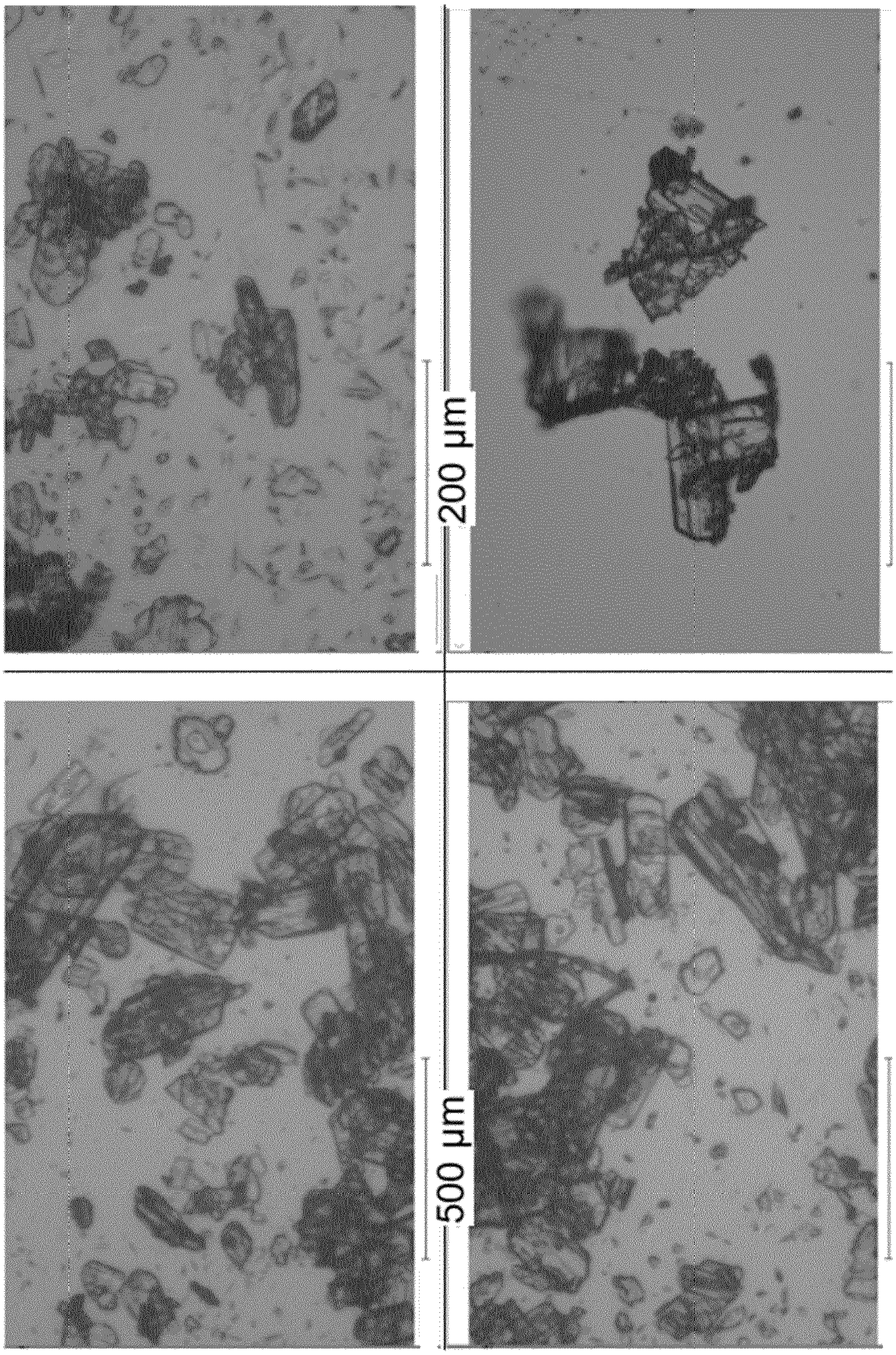

[Fig. 12b]

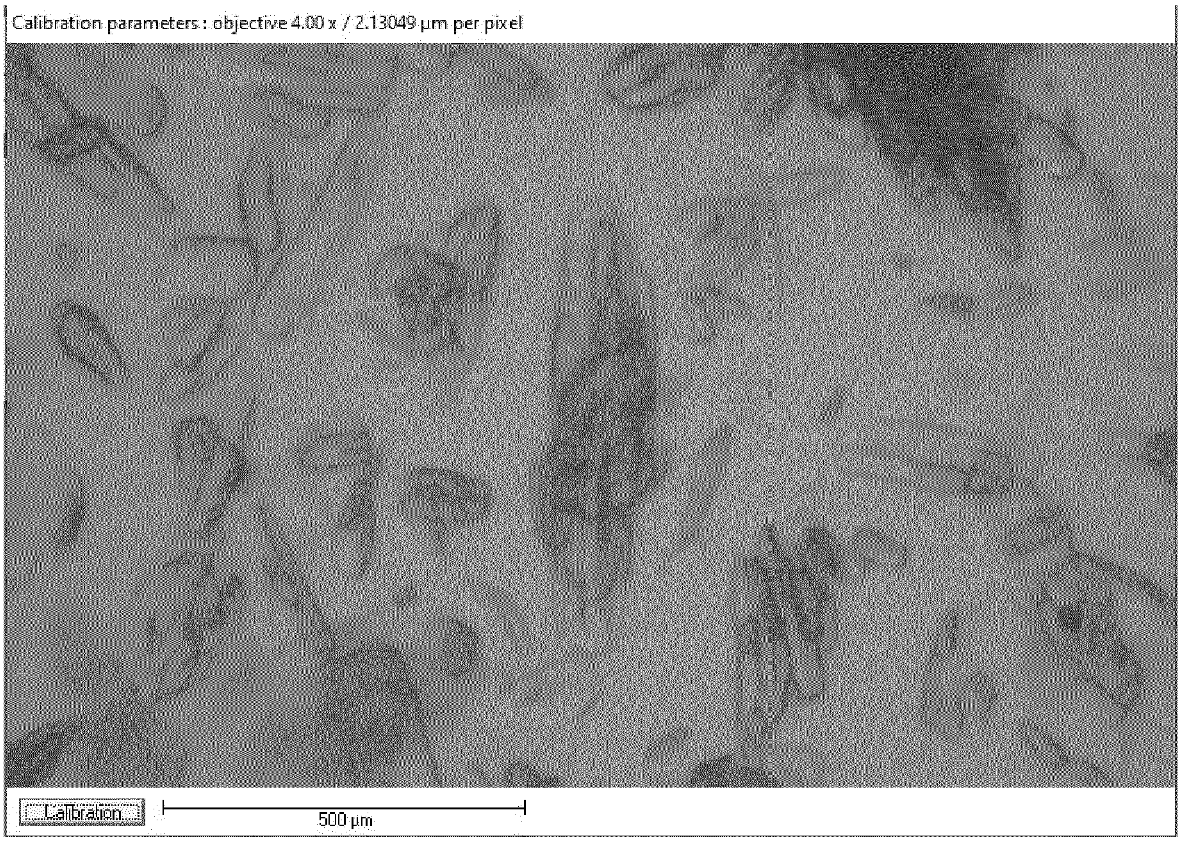


[Fig. 12c]

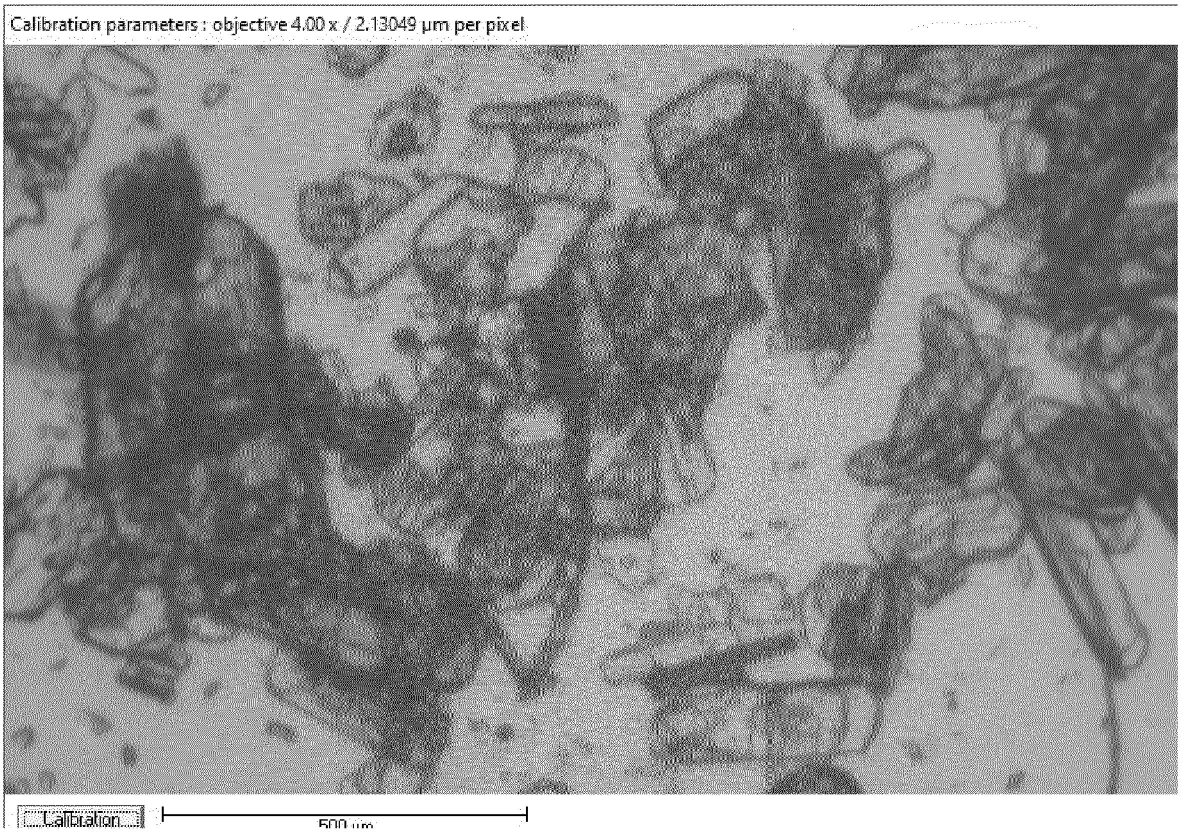

[Fig. 13]

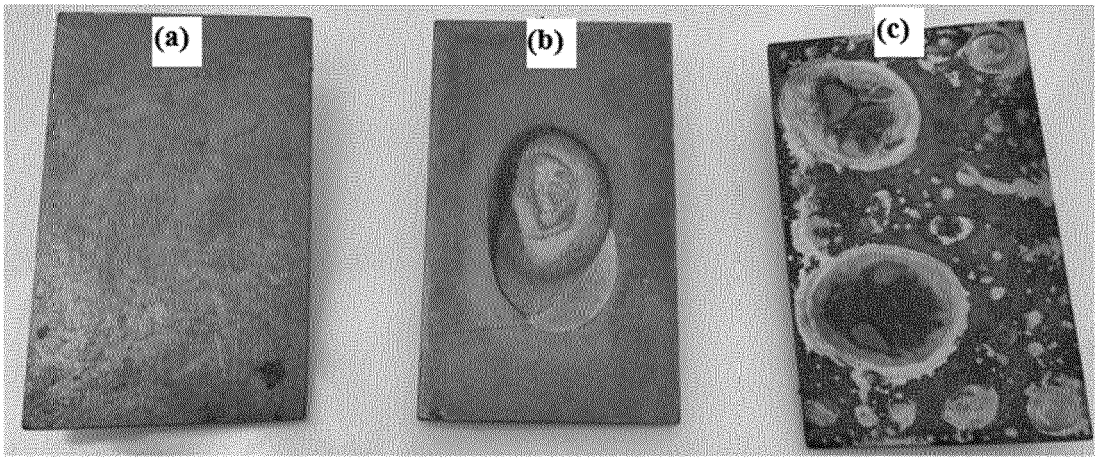


**(a) Control plate**

**(b) Plate in the presence of ATBS according to example 1**

**(c) Plate in the presence of ATBS.Na according to example 2a**

[Fig. 14]

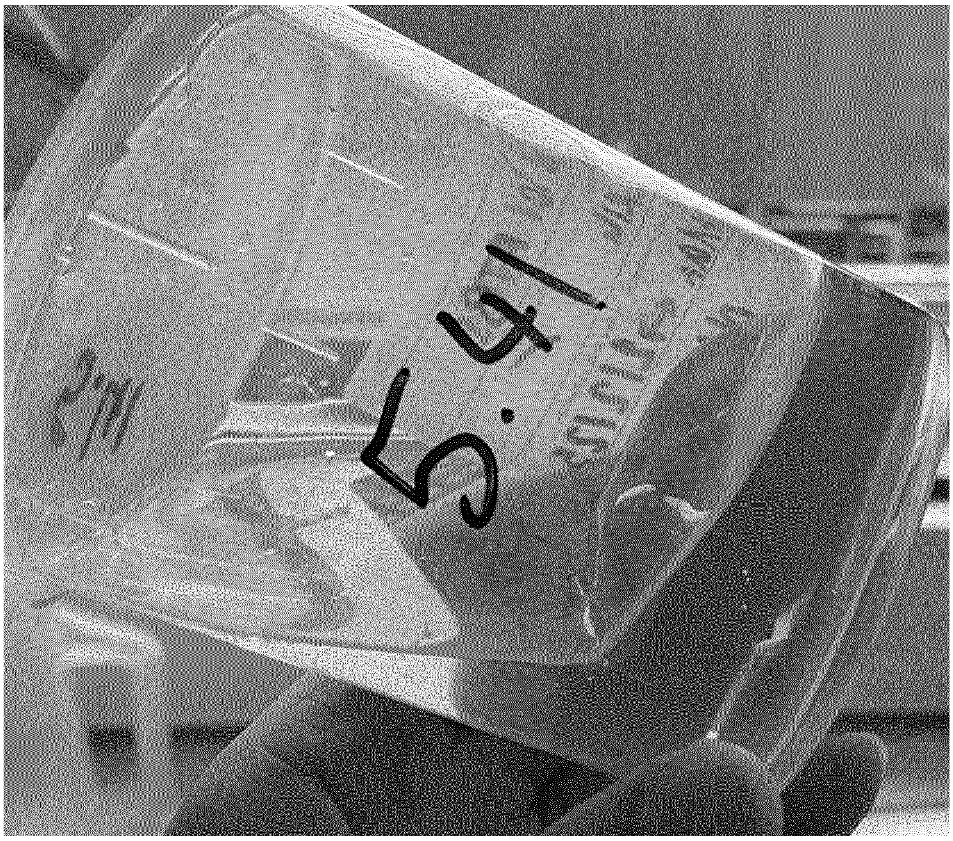

[Fig. 15]

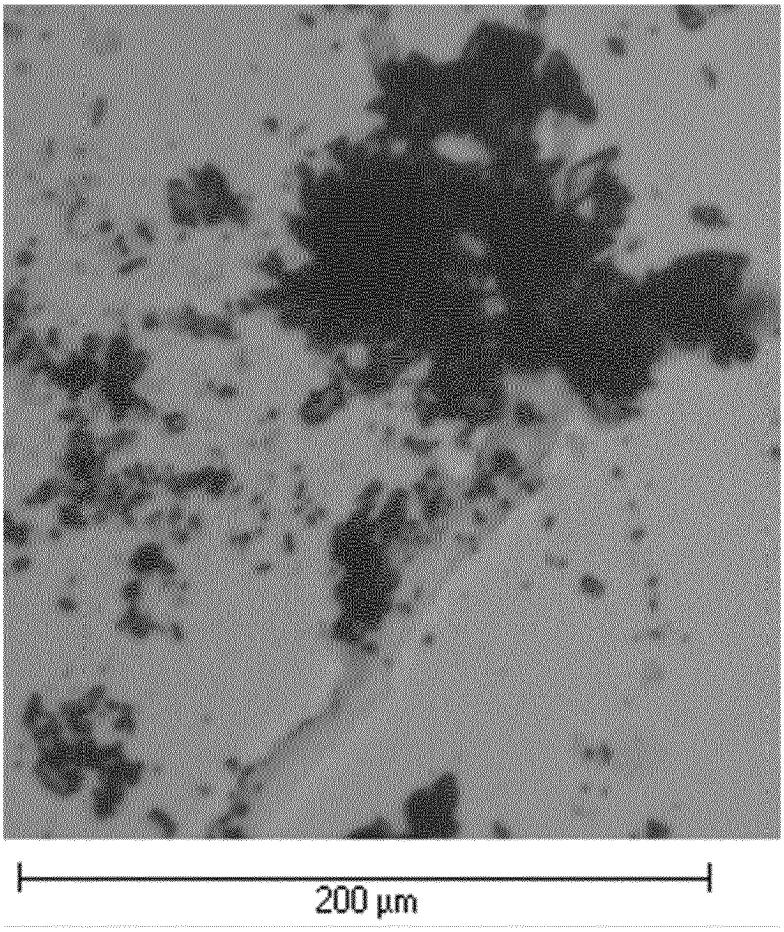
200 µm

[Fig. 16]

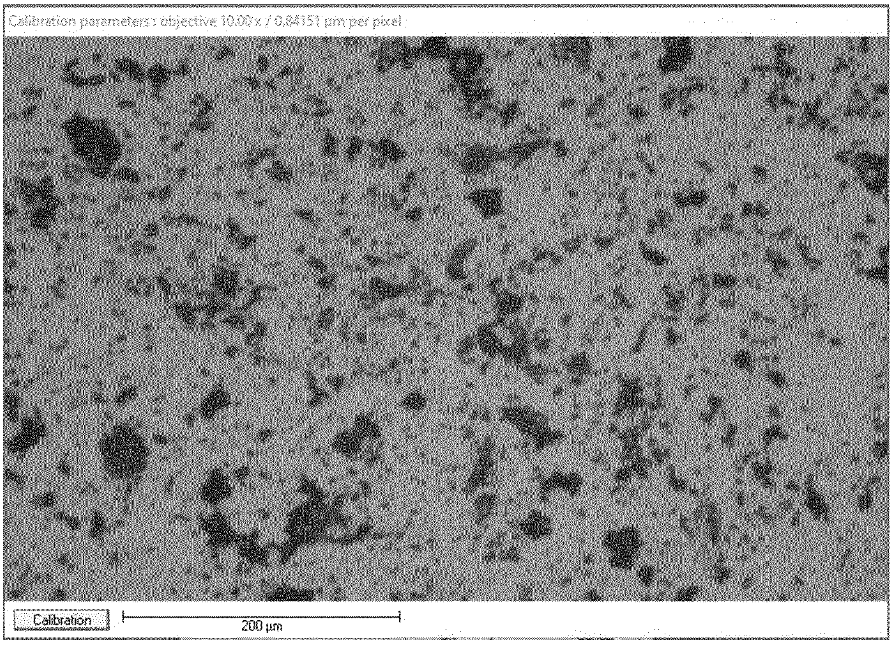
Calibration parameters : objective 10.00 x / 0.84151 µm per pixel
Calibration
200 µm

[Fig. 17]

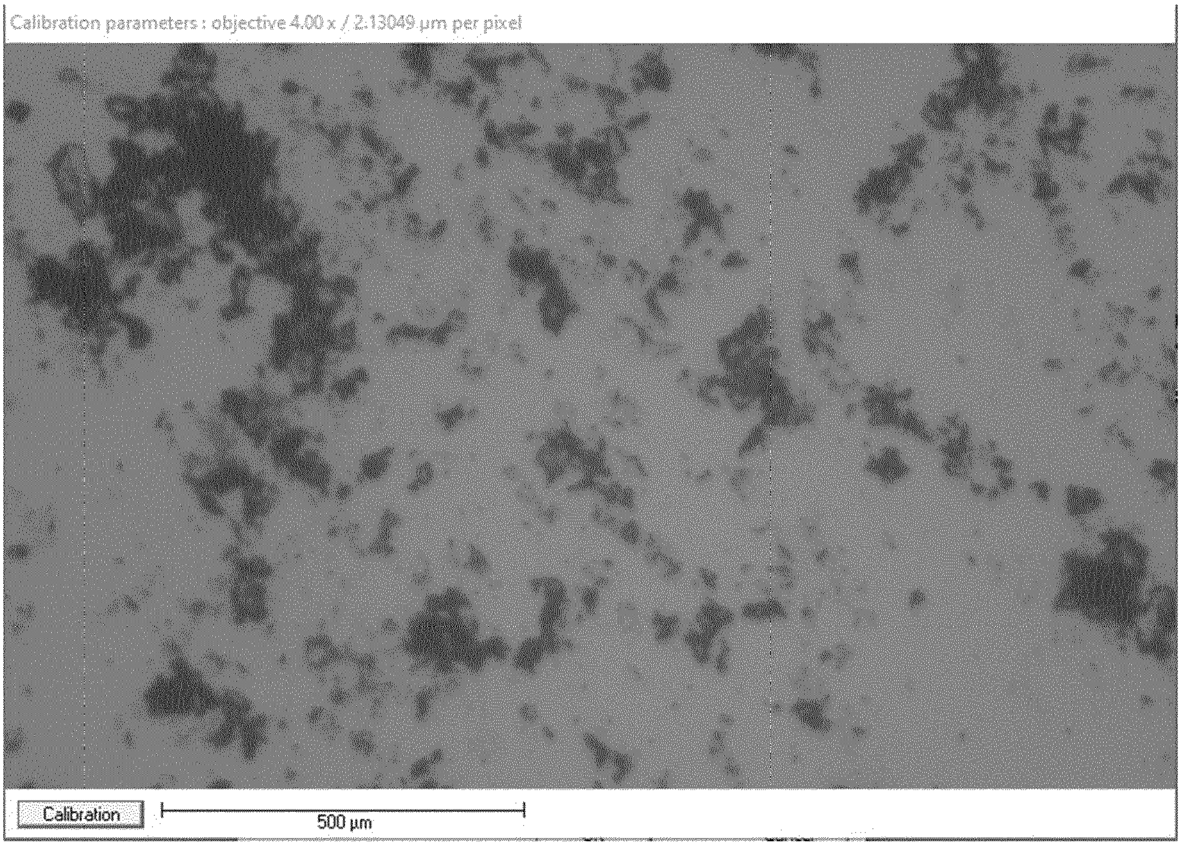
Calibration parameters : objective 4.00 x / 2.13049 µm per pixel
Calibration
500 µm

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6448347 B
- CN 102351744
- WO 2009072480 A
- JP 2008307822 A
- JP 2003137857 A
- WO 2018172676 A
- US 6331647 B1
- WO 2013079507 A2
- WO 2021123599 A
- US 6331647 B
- WO 2013079507 A